# EUROPEAN PATENT APPLICATION

(11) **EP 2 960 330 A1**
(43) Date of publication of application: **30.12.2015**
(21) Application number: 14174722.0
(22) Date of filing: 27.06.2014
(51) Int. Cl.: C12N 9/10

(54) **Glycosyl transferases and their uses**

(71) Applicant: Technische Universität München, 80333 München (DE)
(72) Inventor: Schwab, Wilfried, 97828 Marktheidenfeld (DE)
(74) Representative: Engelhard, Markus

(57) **Abstract**

The present invention relates to novel glycosyl transferases from *Vitis vinifera* that are capable of catalyzing the formation of certain glycosides with high efficiency, to nucleic acid molecules encoding such glycosyl transferases, to vectors, host cells and transgenic plants comprising nucleic acid sequences coding for such glycosyl transferases, and to methods for preparing and uses of such glycosyl transferases.

## Description

The present invention relates to novel glycosyl transferases from *Vitis vinifera* that are capable of catalyzing the formation of certain glycosides with high efficiency, to nucleic acid molecules encoding such glycosyl transferases, to vectors, host cells and transgenic plants comprising nucleic acid sequences coding for such glycosyl transferases, to methods for preparing and to uses of such glycosyl transferases.

Due to their manifold physiological activities, glycosylated natural products are important components of e.g. foodstuffs, pharmaceutical products and cosmetics. Thus, glycosylated diterpenes, steroids and flavonoids are for example used in the food industry as sweeteners (steviosides, glycyrrhizin, neohesperidin, dihydrochalcone) and glycosylated flavonoids as bitterns (neohesperidin), while steroid glycosides and glycosides of antibiotics are employed as medicaments and glycoside extracts as cosmetic products. Moreover, since many terpenes are important fragrance and flavor compounds, the corresponding terpene glycosides can be useful as "slow release" aroma compounds, or can be utilized as antimicrobials, detergents and emulsifier. Glycoside esters of terpene, on the other hand, have antimicrobial activity.

Typically, industrial production of glycosides is carried out by the Koenigs-Knorr process (i.e. organic-chemical substitution of a glycosyl halide with an alcohol to yield a glycoside) or reversed enzymatic hydrolysis or transglycosylation employing glycosidases. However, such methods have a low efficiency, require heavy metal catalysts and anhydrous conditions in the case of chemical synthesis, and thus do not allow to prepare large quantities of glycosides, in particular (mono)terpene glycosides, in a cost-efficient manner.

In nature, regioselective and enantioselective transfer of sugars is catalyzed by nucleoside diphosphate carbohydrate dependent glycosyl transferases, such as UDP-glucose dependent glycosyl transferases (UGTs). Glycosyl transferases of small molecules transfer sugar to a multitude of acceptors, such as antibiotics, lipids, hormones, secondary metabolites or toxins. In plants, a remarkably large array of different small molecules is glycosylated, including terpenoids, alkaloids, cyanohydrins and glucosinolates as well as flavonoids, isoflavonoids, anthocyanidins, phenylpropanoids and phytohormones. The transfer of a carbohydrate group onto a lipophilic acceptor modifies the chemical characteristics and bioactivity of the acceptor and enables it to access membrane transport systems. Some glycosyl transferases are considered highly specific with respect to substrate-, regio- and stereospecificity, whereas others glycosylate a broad range of acceptors (a phenomenon called promiscuity).

Although glycosylation of plant hormones, phenylpropanoids, flavonoids, betalains and coumarins with recombinant UGTs has frequently been described, enzymatic transfer to monoterpenes has been observed quite rarely.

Moreover, glycosyl transferases known from the prior art only have relatively low glycosylation and esterification activities, in particular for the glycosylation of terpenes or monoterpenes and the formation of glycose esters of terpenes or monoterpenes. Thus, the glycosyl transferases available do not allow for cost-efficient preparation of glycosides or glycose esters, in particular of terpene and monoterpene glycosides or terpene and monoterpene glycose esters, and thus do not allow for biotechnological preparation of such glycosides or glycose esters at an industrial scale.

Thus, there is a need in the art for improved ways and reagents for the preparation of glycosides and glycose esters, in particular (mono)terpene glycosides and (mono)terpene glycose esters. Moreover, there is a need in the art for ways and reagents that allow for the preparation of glycosides and glycose esters, in particular (mono)terpene glycosides and (mono)terpene glycose esters, on an industrial scale by biotechnological processes. Moreover, there is a need in the art for glycosyl transferases with high activity for the preparation of glycosides and glycose esters, in particular (mono)terpene glycosides and (mono)terpene glycose esters.

These objects are solved by the below-described aspects of the present invention, in particular by a glycosyl transferase according to claim 1, a nucleic acid molecule according to claim 6, a vector according to claim 7, a host cell according to claim 8, a transgenic plant according to claim 10, the use of a glycosyl transferase according to claim 12, a method of forming a terpene glycoside, terpene glycose ester, octanyl glycoside, furaneyl glycoside or hexanyl glycoside according to claim 13, a method of producing a terpene glycoside, terpene glycose ester, octanyl glycoside, furaneyl glycoside or hexanyl glycoside according to claim 14, and a method of producing a protein having glycosyl transferase activity according to claim 15. Preferred embodiments are defined in the dependent claims and below.

In a first aspect, the present invention relates to a glycosyl transferase having an amino acid sequence that
a) comprises the sequence of SEQ ID NO: 1; or
b) comprises a sequence that is at least 90%, preferably at least 95%, more preferably at least 98%, identical to SEQ ID NO: 1; or
c) comprises a part of the sequence of SEQ ID NO: 1, wherein, preferably, said part of the sequence of SEQ ID NO: 1 is at least 50, preferably at least 80, more preferably at least 100, more preferably at least 200, amino acids in length; or
d) comprises a sequence that is at least 90%, preferably at least 95%, more preferably at least 98%, identical to a part of the sequence of SEQ ID NO: 1, wherein, preferably, said part of the sequence of SEQ ID NO: 1 is at least 50, preferably at least 80, more preferably at least 100, more preferably at least 200, amino acids in length;
e) comprises the sequence of SEQ ID NO: 2; or
f) comprises a sequence that is at least 90%, preferably at least 95%, more preferably at least 98%, identical to SEQ ID NO: 2; or
g) comprises a part of the sequence of SEQ ID NO: 2, wherein, preferably, said part of the sequence of SEQ ID NO: 2 is at least 50, preferably at least 80, more preferably at least 100, more preferably at least 200, amino acids in length; or
h) comprises a sequence that is at least 90%, preferably at least 95%, more preferably at least 98%, identical to a part of the sequence of SEQ ID NO: 2, wherein, preferably, said part of the sequence of SEQ ID NO: 2 is at least 50, preferably at least 80, more preferably at least 100, more preferably at least 200, amino acids in length.

In some embodiments, said glycosyl transferase is a small molecule glycosyl transferase.

In some embodiments, the glycosyl transferase is a terpene glycosyl transferase, preferably a monoterpene glycosyl transferase, more preferably a UDP-glucose:monoterpene P-D-glucosyltransferase.

In some embodiments, said glycosyl transferase is capable of using UDP-glucose as sugar donor. Preferably, said glycosyl transferase uses UDP-glucose more efficiently as sugar donor than UDP-xylose, UDP-glucuronic acid, UDP-arabinose, UDP-rhamnose, UDP-galactose, GDP-fucose, GDP-mannose and/or CMP-sialic acid, as seen by radiochemical analysis. In such radiochemical analysis, individual reactions are carried out in which different radiolabelled sugar donors (such as radiolabelled UDP-glucose, UDP-xylose and UDP-glucuronic acid) that carry a radionuclide in their sugar group are reacted under appropriate conditions and in the presence of the glycosyl transferase with a certain acceptor molecule. By comparing the amount of radiolabel that was transferred from the different sugar donors to the acceptor molecule, it can be determined which sugar donor the glycosyl transferase uses more efficiently than the others.

In some embodiments, the glycosyl transferase is capable of catalyzing transfer of a sugar group from a sugar donor to a hydroxyl group of a hydroxy-containing terpene and/or a carboxyl group of a carboxy-containing terpene.

In some embodiments, the glycosyl transferase is capable of catalyzing formation of a glycoside in which a sugar is linked to a hydroxy-containing terpene through a β-D-glycosyl linkage and/or formation of a glycose ester in which a sugar is linked to a carboxy-containing terpene through a β-D-glycose ester linkage.

In some embodiments, the glycosyl transferase is capable of catalyzing glycosylation, preferably glucosylation, of geraniol, (R-)linalool, (R- and/or S-)citronellol, nerol, hexanol and/or octanol, preferably geraniol and/or (R- and/or S-)citronellol, wherein, preferably, said glycosyl transferase has an amino acid sequence that
a) comprises the sequence of SEQ ID NO: 1; or
b) comprises a sequence that is at least 90%, preferably at least 95%, more preferably at least 98%, identical to SEQ ID NO: 1; or
c) comprises a part of the sequence of SEQ ID NO: 1, wherein, preferably, said part of the sequence of SEQ ID NO: 1 is at least 50, preferably at least 80, more preferably at least 100, more preferably at least 200, amino acids in length; or
d) comprises a sequence that is at least 90%, preferably at least 95%, more preferably at least 98%, identical to a part of the sequence of SEQ ID NO: 1, wherein, preferably, said part of the sequence of SEQ ID NO: 1 is at least 50, preferably at least 80, more preferably at least 100, more preferably at least 200, amino acids in length.

In some embodiments, the glycosyl transferase is capable of catalyzing glycosylation, preferably glucosylation, of furaneol, wherein, preferably, said glycosyl transferase has an amino acid sequence that
a) comprises the sequence of SEQ ID NO: 1; or
b) comprises a sequence that is at least 90%, preferably at least 95%, more preferably at least 98%, identical to SEQ ID NO: 1; or
c) comprises a part of the sequence of SEQ ID NO: 1, wherein, preferably, said part of the sequence of SEQ ID NO: 1 is at least 50, preferably at least 80, more preferably at least 100, more preferably at least 200, amino acids in length; or
d) comprises a sequence that is at least 90%, preferably at least 95%, more preferably at least 98%, identical to a part of the sequence of SEQ ID NO: 1, wherein, preferably, said part of the sequence of SEQ ID NO: 1 is at least 50, preferably at least 80, more preferably at least 100, more preferably at least 200, amino acids in length.

In some embodiments, the glycosyl transferase is not capable of catalyzing glycosylation, preferably glucosylation, of cyanidin, pelargonodin, quercetin and/or kaempferol, wherein, preferably, said glycosyl transferase has an amino acid sequence that
a) comprises the sequence of SEQ ID NO: 1; or
b) comprises a sequence that is at least 90%, preferably at least 95%, more preferably at least 98%, identical to SEQ ID NO: 1; or
c) comprises a part of the sequence of SEQ ID NO: 1, wherein, preferably, said part of the sequence of SEQ ID NO: 1 is at least 50, preferably at least 80, more preferably at least 100, more preferably at least 200, amino acids in length; or
d) comprises a sequence that is at least 90%, preferably at least 95%, more preferably at least 98%, identical to a part of the sequence of SEQ ID NO: 1, wherein, preferably, said part of the sequence of SEQ ID NO: 1 is at least 50, preferably at least 80, more preferably at least 100, more preferably at least 200, amino acids in length.

In some embodiments, the glycosyl transferase is capable of catalyzing glycosylation, preferably glucosylation, of geraniol, (R- and/or S-)citronellol, nerol, hexanol, octanol, 8-hydroxylinalool, *trans* 2-hexenol, and/or farnesol, preferably geraniol, wherein, preferably, said glycosyl transferase has an amino acid sequence that
d) comprises a sequence that is at least 90%, preferably at least 95%, more preferably at least 98%, identical to a part of the sequence of SEQ ID NO: 2, wherein, preferably, said part of the sequence of SEQ ID NO: 2 is at least 50, preferably at least 80, more preferably at least 100, more preferably at least 200, amino acids in length;
e) comprises the sequence of SEQ ID NO: 2; or
f) comprises a sequence that is at least 90%, preferably at least 95%, more preferably at least 98%, identical to SEQ ID NO: 2; or
g) comprises a part of the sequence of SEQ ID NO: 2, wherein, preferably, said part of the sequence of SEQ ID NO: 2 is at least 50, preferably at least 80, more preferably at least 100, more preferably at least 200, amino acids in length; or
h) comprises a sequence that is at least 90%, preferably at least 95%, more preferably at least 98%, identical to a part of the sequence of SEQ ID NO: 2, wherein, preferably, said part of the sequence of SEQ ID NO: 2 is at least 50, preferably at least 80, more preferably at least 100, more preferably at least 200, amino acids in length.

In some embodiments, the glycosyl transferase is not capable of catalyzing glycosylation, preferably glucosylation, of cyanidin, pelargonodin, quercetin, kaempferol, linalool, terpineol, benzyl alcohol, phenyl ethanol, eugenol, mandelonitrile, 3-methyl-2-butenol, 3-methyl-3-butenol, cis-3-hexenol and/or furaneol, wherein, preferably, said glycosyl transferase has an amino acid sequence that
d) comprises a sequence that is at least 90%, preferably at least 95%, more preferably at least 98%, identical to a part of the sequence of SEQ ID NO: 2, wherein, preferably, said part of the sequence of SEQ ID NO: 2 is at least 50, preferably at least 80, more preferably at least 100, more preferably at least 200, amino acids in length;
e) comprises the sequence of SEQ ID NO: 2; or
f) comprises a sequence that is at least 90%, preferably at least 95%, more preferably at least 98%, identical to SEQ ID NO: 2; or
g) comprises a part of the sequence of SEQ ID NO: 2, wherein, preferably, said part of the sequence of SEQ ID NO: 2 is at least 50, preferably at least 80, more preferably at least 100, more preferably at least 200, amino acids in length; or
h) comprises a sequence that is at least 90%, preferably at least 95%, more preferably at least 98%, identical to a part of the sequence of SEQ ID NO: 2, wherein, preferably, said part of the sequence of SEQ ID NO: 2 is at least 50, preferably at least 80, more preferably at least 100, more preferably at least 200, amino acids in length.

In some embodiments, the glucosyl transferase activity (k_{cat}/K_{M}) of said glycosyl transferase for geraniol, citronellol and/or nerol is higher than the glucosyl transferase activity of said glycosyl transferase for benzyl alcohol by at least a factor of 2. In some embodiments, the glucosyl transferase activity (k_{cat}/K_{M}) of said glycosyl transferase for geraniol is higher than the glucosyl transferase activity of said glycosyl transferase for hexanol by at least a factor of 4.

In some embodiments, said glycosyl transferase has a glucosyl transferase activity (k_{cat}/K_{M}) for geraniol, that is by at least a factor of 2, preferably by at least a factor of 5, more preferably by at least a factor of 10, more preferably by at least a factor of 20, more preferably by at least a factor of 30, more preferably by at least a factor of 40 higher than the glucosyl transferase activity of the glycosyl transferase UGT85B1 of *Sorghum bicolor* for geraniol.

In some embodiments, said glycosyl transferase has a glucosyl transferase activity (k_{cat}/K_{M}) for nerol that is by at least a factor of 2, preferably by at least a factor of 3, more preferably by at least a factor of 4, more preferably by at least a factor of 5 higher than the glucosyl transferase activity of the glycosyl transferase UGT85B1 of *Sorghum bicolor* for nerol.

In some embodiments, said glycosyl transferase has a glucosyl transferase activity (k_{cat}/K_{M}) for citronellol that is by at least a factor of 2, preferably by at least a factor of 3 higher than the glucosyl transferase activity of the glycosyl transferase UGT85B1 of *Sorghum bicolor* for citronellol.

In some embodiments, said glycosyl transferase can be expressed more efficiently as a recombinant protein in *E.coli* cells than the glycosyl transferase UGT85B1 of *Sorghum bicolor.*

In a second aspect, the present invention relates to an (isolated) nucleic acid molecule encoding a glycosyl transferase as defined in any of the embodiments described above, wherein, preferably, said nucleic acid molecule is a DNA molecule.

In a third aspect, the present invention relates to a vector comprising a DNA sequence encoding a glycosyl transferase as defined in any of the embodiments described above.

Preferably, said vector is an expression vector, preferably an expression vector for expression of a glycosyl transferase as defined in any of the embodiments described above.

In a fourth aspect, the present invention relates to a host cell containing or transfected with the nucleic acid molecule according to the second aspect of the invention described above or the vector according to the third aspect of the invention described above, wherein, preferably, said host cell is not a cell of *Vitis vinifera,* more preferably not a cell of a grape vine, and/or wherein, preferably, said host cell is a non-human cell, preferably a bacterial cell, more preferably an *E. coli* cell.

In some embodiments, said host cell produces/expresses a glycosyl transferase as defined in any of the embodiments above.

In a fifth aspect, the present invention relates to a transgenic plant comprising a nucleic acid molecule as defined above or a vector as defined above, wherein, preferably, said plant is not a *Vitis vinifera* plant, more preferably not a grape vine.

In some embodiments, said transgenic plant produces/expresses a glycosyl transferase as defined in any of the embodiments above.

In a sixth aspect, the present invention relates to the use of a glycosyl transferase as defined in any of the embodiments above or a nucleic acid molecule as defined in the second aspect of the invention or a vector as defined in the third aspect of the invention or a host cell as defined in any of the embodiments above or a transgenic plant as defined in any of the embodiments above for producing a terpene glycoside, terpene glycose ester, octanyl glycoside, furaneyl glycoside or hexanyl glycoside.

Preferably, said terpene glycoside is selected from the group consisting of geranyl β-D-glucoside, (R-)linaloyl β-D-glucoside, (R- and/or S-)citronellyl β-D-glucoside, neryl β-D-glucoside, 8-hydroxylinaloyl glucoside and farnesyl glucoside, more preferably said terpene glycoside is geranyl β-D-glucoside and/or (R- and/or S-)citronellyl β-D-glucoside, more preferably said terpene glycoside is geranyl β-D-glucoside.

Preferably, said octanyl glycoside is octanyl glucoside. Preferably, said furaneyl glycoside is furaneyl glucoside. Preferably, said hexanyl glycoside is hexanyl glucoside.

Preferably, said production of said terpene glycoside, terpene glycose ester, octanyl glycoside, furaneyl glycoside or hexanyl glycoside does not involve steps carried out *in vivo.* Preferably, said production of said terpene glycoside, terpene glycose ester, octanyl glycoside, furaneyl glycoside or hexanyl glycoside is carried out in a host cell or transgenic plant as defined above, preferably in an *E.coli* cell.

In a seventh aspect, the present invention relates to a method of forming
a) a terpene glycoside in which a hydroxy-containing terpene is covalently linked to a sugar group through a glycosidic bond or
b) a terpene glycose ester in which a carboxy-containing terpene is covalently linked to a sugar group through a glycose ester bond or
c) an octanyl glycoside in which octanol is covalently linked to a sugar group through a glycosidic bond or
d) a furaneyl glycoside in which furaneol is covalently linked to a sugar group through a glycosidic bond or
e) a hexanyl glycoside in which hexanol is covalently linked to a sugar group through a glycosidic bond,
said method comprising contacting
a) a hydroxy-containing terpene or
b) a carboxy-containing terpene or
c) octanol or
d) furaneol or
e) hexanol
with a sugar donor and a glycosyl transferase as defined in any of the embodiments above under conditions appropriate for the transfer of the sugar group of said sugar donor to
a) a hydroxyl group of said hydroxy-containing terpene or
b) a carboxyl group of said carboxy-containing terpene or
c) the hydroxyl group of octanol or
d) the hydroxyl group of furaneol or
e) the hydroxyl group of hexanol
under formation of
a) a glycosidic bond between said terpene and said sugar group or
b) a ester bond between said terpene and said sugar group or
c) a glycosidic bond between octanol and said sugar group or
d) a glycosidic bond between furaneol and said sugar group or
e) a glycosidic bond between hexanol and said sugar group,
thereby forming
a) a terpene glycoside or
b) a terpene glycose ester or
c) an octanyl glycoside or
d) a furaneyl glycoside or
e) a hexanyl glycoside.

Preferably, said glycosyl transferase is a recombinantly expressed glycosyl transferase.

Preferably, said terpene glycoside is selected from the group consisting of geranyl β-D-glucoside, (R-)linaloyl β-D-glucoside, (R- and/or S-)citronellyl β-D-glucoside, neryl β-D-glucoside, 8-hydroxylinaloyl glucoside and farnesyl glucoside,
said sugar group is glucose and said sugar donor is UDP-glucose,
and said hydroxy-containing terpene is selected from the group consisting of geraniol, (R-) linalool, (R- and/or S-)citronellol, nerol, 8-hydroxylinalool and farnesol.

More preferably, said terpene glycoside is geranyl β-D-glucoside or (R- and/or S-)citronellyl β-D-glucoside,
said sugar group is glucose and said sugar donor is UDP-glucose,
and said hydroxy-containing terpene is geraniol or (R- and/or S-)citronellol.

More preferably, said terpene glycoside is geranyl β-D-glucoside,
said sugar group is glucose and said sugar donor is UDP-glucose,
and said hydroxy-containing terpene is geraniol.

Preferably, said sugar group is glucose, said sugar donor is UDP-glucose, and said octanyl glycoside is octanyl glucoside. Preferably, said sugar group is glucose, said sugar donor is UDP-glucose, and said furaneyl glycoside is furaneyl glucoside. Preferably, said sugar group is glucose, said sugar donor is UDP-glucose, and said hexanyl glycoside is hexanyl glucoside.

In some embodiments, said method is an *in vitro* method which, preferably, does not involve any steps carried out *in vivo.* In some embodiments, said method is an *in vivo* method carried out in a host cell or transgenic plant as defined above, more preferably an *in vivo* method carried out in *E.coli.*

In an eighth aspect, the present invention relates to a method of producing a terpene glycoside, terpene glycose ester, octanyl glycoside, furaneyl glycoside or hexanyl glycoside, said method comprising the steps of:
- culturing or growing a host cell as defined in any of the embodiments above or a transgenic plant as defined in any of the embodiments above; and
- collecting from said host cell or transgenic plant said terpene glycoside, terpene glycose ester, octanyl glycoside, furaneyl glycoside or hexanyl glycoside.

Preferably, said terpene glycoside is a terpene glycoside in which a hydroxy-containing terpene is covalently linked to a sugar group. Preferably, said terpene glycose ester is a terpene glycose ester in which a carboxy-containing terpene is covalently linked to a sugar group.

In some embodiments, said hydroxy-containing terpene is a monoterpene, preferably geraniol, (R-)linalool, (R- and/or S-)citronellol, nerol, 8-hydroxylinalool or farnesol, more preferably geraniol or citronellol, more preferably geraniol, and/or said sugar group is a glucosyl group.In some embodiments, said terpene glycoside is selected from the group consisting of geranyl β-D-glucoside, (R-)linaloyl β-D-glucoside, (R- and/or S-)citronellyl β-D-glucoside, neryl β-D-glucoside, 8-hydroxylinaloyl glucoside and farnesyl glucoside, preferably geranyl β-D-glucoside or (R- and/or S-)citronellyl β-D-glucoside, more preferably geranyl β-D-glucoside.

In some embodiments, said octanyl glycoside is octanyl glucoside. In some embodiments, said furaneyl glycoside is furaneyl glucoside. In some embodiments, said hexanyl glycoside is hexanyl glucoside.

Preferably, during said culturing or growing said host cell or transgenic plant said hydroxy-containing terpene is present in said host cell or transgenic plant. Preferably, during said culturing or growing said host cell or transgenic plant said carboxy-containing terpene is present in said host cell or transgenic plant. Preferably, during said culturing or growing said host cell or transgenic plant octanol is present in said host cell or transgenic plant. Preferably, during said culturing or growing said host cell or transgenic plant furaneol is present in said host cell or transgenic plant. Preferably, during said culturing or growing said host cell or transgenic plant hexanol is present in said host cell or transgenic plant. Preferably, during said culturing or growing said host cell or transgenic plant UDP-glucose is present in the culture medium used for culturing said host cell or in the water used for watering that transgenic plant.

In some embodiments, said culturing or growing said host cell is carried out in a bioreactor.

In a ninth aspect, the present invention relates to a method of producing a protein having glycosyl transferase activity and/or enzymatic activity for the catalysis of glycose esterification, said method comprising the steps of:
- culturing or growing a host cell as defined in any of the embodiments above or a transgenic plant as defined in any of the embodiments above; and, preferably,
- collecting from the host cell or transgenic plant a protein having glycosyl transferase activity and/or enzymatic activity for the catalysis of glycose esterification.

Preferably, said glycosyl transferase activity is an activity of transferring the sugar group of a sugar donor to a hydroxyl group of a hydroxy-containing terpene under formation of a glycosidic bond between said hydroxy-containing terpene and said sugar group.

Preferably, said hydroxy-containing terpene is a monoterpene, preferably geraniol, (R-)linalool, (R- and/or S-)citronellol, nerol, 8-hydroxylinalool or farnesol, more preferably geraniol or citronellol, more preferably geraniol, and/or said sugar donor is UDP-glucose. Preferably, said glycosyl transferase activity is an activity of transferring the sugar group of a sugar donor to a hydroxyl group of octanol, furaneol or hexanol under formation of a glycosidic bond between said octanol, furaneol or hexanol and said sugar group.

Preferably, said sugar donor is UDP-glucose and said sugar group is a glucosyl group.

Preferably, said enzymatic activity for the catalysis of glycose esterification is an activity of transferring the sugar group of a sugar donor to a carboxyl group of a carboxy-containing terpene under formation of a glycose ester bond between said carboxy-containing terpene and said sugar group.

Preferably, said protein having glycosyl transferase activity and/or enzymatic activity for the catalysis of glycose esterification is a glycosyl transferase as defined in any of the embodiments above.A "glycosyl transferase" in an enzyme of EC class 2.4 that catalyzes the transfer of a monosaccharide moiety from a sugar donor to an acceptor molecule under formation of a glycosidic linkage between the sugar (the glycone) and the acceptor molecule (the aglycone) (see, e.g., Bowles et al., 2006). The sugar donor is typically an activated sugar precursor and can be, for example, UDP(uridine diphosphate)-glucose wherein the sugar is glucose, UDP-xylose wherein the sugar is xylose, UDP-glucuronic acid wherein the sugar is glucuronic acid, UDP-arabinose wherein the sugar is arabinose, UDP-rhamnose wherein the sugar is rhamnose, UDP-galactose wherein the sugar is galactose, GDP(guanosin diphosphate)-fucose wherein the sugar is fucose, GDP-mannose wherein the sugar is mannose or CMP(cytidine monophosphate)-sialic acid wherein the sugar is sialic acid. If the glycosyl transferase is a glucosyl transferase, then the sugar donor is UDP-glucose. The acceptor molecule may be an alcohol, such as the alcohol of a terpenoid, alkaloid, cyanohydrin, glucosinolate, flavonoid, isoflavonoid, anthocyanidin, phenylpropanoid, polyphenol, hydroquinone, amine, carbohydrate (monomeric or oligomeric), fatty acid or lipids. Examples of glycosyl transferases are UDP-glucosyltransferases, UDP-arabinosyltransferases, UDP-glucuronosyltransferases, UDP-xylosyltransferases, UDP-galactosyltransferases, UDP-rhamnosyltransferases, GDP-fucosyltransferase, GDP-mannosyltransferase, or CMP-sialyltransferase. A glycosyl transferase may or may not have an additional enzymatic activity for the catalysis of glycose esterification, i.e. for transferring the sugar group of a sugar donor to a carboxyl group of a carboxy-containing acceptor molecule under formation of a glycose ester bond between said carboxy-containing acceptor molecule and said sugar group.

A "small molecule glycosyl transferase" is a glycosyl transferase that catalyzes the transfer of a monosaccharide moiety from a sugar donor to a small molecule as acceptor molecule. A small molecule is a molecule that has a molecular weight below 1 500 Dalton, preferably below 1 000 Dalton. A "terpene glycosyl transferase" is a glycosyl transferase that catalyzes the transfer of a monosaccharide moiety from a sugar donor to a terpene as acceptor molecule. A "monoterpene glycosyl transferase" is a glycosyl transferase that catalyzes the transfer of a monosaccharide moiety from a sugar donor to a monoterpene as acceptor molecule. An "UDP-glucose:monoterpene β-D-glucosyltransferase" is a glycosyl transferase that catalyzes the transfer of a glucose moiety from a UDP-glucose as sugar donor to a monoterpene as acceptor molecule under formation of covalent a β-D-glycosidic bond.

At some instance, the present invention refers to a glycosyl transferase "having" a certain amino acid sequence. This is meant to designate that the amino acid sequence of said glycosyl transferase consists of said certain amino acid sequence, i.e. the glycosyl transferase comprises only said certain amino acid sequence and no further amino acid sequence(s) beyond said certain amino acid sequence.

Glycosyl transferases having an amino acid sequence comprising SEQ ID NO: 1 (i.e. the sequence of VvGT14) or SEQ ID NO: 2 (i.e. the sequence of VvGT15) or a related amino acid sequence can be obtained by standard methods of recombinant DNA technology, for example as described in Example 1 below.

If the present application refers to a specific allelic/splice form of VvGT14 by the general designation "VvGT14", this is meant to refer to VvGT14a. If the present application refers to a specific allelic/splice form of VvGT15 by the general designation "VvGT15 ", this is meant to refer to VvGT15a.

A "glycoside", as used herein, is a molecule in which a sugar (the "glycone" part or "glycone component" of the glycoside) is bonded to a non-sugar (the "aglycone" part or "aglycone component") via a glycosidic bond. Accordingly, a glycoside may consist of a sugar as glycone component (designated "Z" in the general chemical structure below) linked through its anomeric carbon atom to the hydroxy group of an alcohol (chemical structure R-OH) as aglycone component, thus resulting in a glycoside of the general chemical structure R-O-Z. For example, in the glycoside linaloyl β-D-glucoside, the glycone component glucose is linked to the aglycone component linalool.

A glycoside can be produced by carrying out a reaction in which an aglycone component (such as a terpene, for example geraniol or citronellol) is mixed under appropriate conditions with a sugar donor (an activated sugar precursor such as UDP-glucose or UDP-glucuronic acid, preferably UDP-glucose) in the presence of a glycosyl transferase as enzymatic catalyzer. For example, 100 µL purified enzyme (50 µg), 100-150 µL Tris-HCl buffer (100 mM, pH 7.5, 10 mM 2-mercaptoethanol), 37 pmol UDP-glucose and 50 µg substrate (dissolved in methyl-tert-butylether) can be incubated at 30°C for 24 hr. This results in the formation of glycosides composed of an aglycone component linked to a glycone component. The glycoside can subsequently be isolated from the reaction mixture by standard methods of extraction and chromatography (see also Example 1).

Alternatively, a glycoside can be produced by culturing or growing a host cell or transgenic plant expressing a glycosyl transferase according to the invention. During culture/growth, such a host cell or transgenic plant will generate glycosides. The glycoside(s) generated in such a host cell or transgenic plant can subsequently be collected from said host cell or transgenic plant by standard methods of extraction and/or chromatography (such as solvent extraction, solid phase extraction and reversed phase chromatography). In such a method for producing a glycoside, the present application may indicate that during said culturing or growing a host cell or transgenic plant a certain compound or substrate (such as the aglycone component) used for formation of the glycoside "is present in said host cell or transgenic plant". This means that the compound or substrate is either produced by said host cell or transgenic plant such that it is present in said host cell/in the cells of said transgenic plant, or that it is added to the host cell or transgenic plant in such a manner that it is taken up by the host cell or transgenic plant and enters into the host cell/cells of the transgenic plant. This may for example be achieved by including the compound or substrate to the culture medium used for culturing the host cells (for example the growth medium used for culturing *E.coli* cells) or, in the case of a transgenic plant, by adding the compound or substrate to the water used for watering the plant (for example, an aqueous solution containing the compound/substrate or an aqueous solution with a low content of ethanol containing the compound/substrate may be added to the culture medium used for culturing the host cells or to the water used for watering the plant).

If the present application refers to "collecting" a certain glycoside, glycose ester or protein from a host cell or transgenic plant, this is meant to designate that said glycoside, glycose ester or protein is separated and/or isolated from other components of said host cell or transgenic plant. This can be achieved by standard methods of extraction and chromatography known to a person of skill in the art (see e.g. Example 1).

As used herein, a "bioreactor" is a vessel in which a (bio)chemical process is carried out which involves organisms (such as host cells) or biochemically active substances derived from such organisms.

A "terpene", as used herein, is a hydrocarbon having a carbon skeleton formally derived by combination of several isoprene units. The term includes hydrocarbons having a carbon skeleton formally derived by combination of several isoprene units covalently linked to at least one hydroxy group, preferably covalently linked to one hydroxy group and/or covalently linked to at least one carboxyl group, preferably covalently linked to one carboxyl group. In some embodiments, the term "terpene" also includes hydrocarbons having a carbon skeleton formally derived by combination of several isoprene units in which up to three, preferably up to two, more preferably one, methyl groups have been moved or removed. In other embodiments, the term "terpene" does not include hydrocarbons having a carbon skeleton formally derived by combination of several isoprene units in which methyl groups have been moved or removed. In some embodiments, the term "terpene" also includes hydrocarbons having a carbon skeleton formally derived by combination of several isoprene units which comprise up to three, preferably up to two, more preferably one, oxygen atom. In some embodiments the term "terpene" does not include hydrocarbons having a carbon skeleton formally derived by combination of several isoprene units which comprise additional oxygen atoms besides the oxygen atom that is part of the above-mentioned hydroxy group. In some embodiments the term "terpene" does not include compounds comprising more than one oxygen atom.

As used herein, a "hydroxy-containing terpene" is a terpene that comprises one or more, preferably one, hydroxy group. As used herein, a "carboxy-containing terpene" is a terpene that comprises one or more, preferably one, carboxyl group. The term "terpene glycoside" refers to a glycoside the aglycone component of which is a terpene. In some embodiments, the term does not include glycosides the aglycone component of which is a terpene in which methyl groups have been moved or removed compared to a terpene formally derived from isoprene units. In some embodiments, the term does not include glycosides the aglycone component of which includes further functional groups in addition to the functional group forming the linkage to the glycone component of the terpene glycoside. In some embodiments, the term does not include glycosides the aglycone component of which includes further oxygen atoms in addition to oxygen atom(s) that are part of the functional group forming the linkage to the glycone component of the terpene glycoside. The term "monoterpene glycoside" refers to a glycoside the aglycone component of which is a monoterpene (formally comprising two isoprene units, such as geraniol, citronellol or linalool). The term "sesquiterpene glycoside" refers to a glycoside the aglycone component of which is a terpene formally comprising three isoprene units (such as farnesol). The term "diterpene glycoside" refers to a glycoside the aglycone component of which is a diterpene (formally comprising four isoprene units, such as steviol).

A "glycose ester", as used herein, is a molecule in which an aglycone component is linked via an ester bond to a sugar component, preferably to a monosaccharide. A "terpene glycose ester" is a glycose ester the aglycone component of which is a terpene.

"Glycose esterification", as used herein, refers to a reaction in which a molecule is linked to a sugar, preferably a monosaccharide, under formation of an ester bond between said molecule and said sugar.

At some instances, the present application refers to a glycosyl transferase being "capable of catalyzing" a certain reaction. For example, the present application may state that a glycosyl transferase is capable of catalyzing transfer of a sugar group from a sugar donor to a certain acceptor. This is meant to designate that under appropriate reaction conditions the rate at which the reaction product (in the example the adduct of the sugar group and the acceptor) is formed is at least 10-fold higher in the presence of said glycosyl transferase than the rate at which the reaction product is formed in a control experiment in the absence of said glycosyl transferase.

At some instances, the present application indicates that a certain glycosyl transferase "has a glucosyl transferase activity" for a substrate A that is "by at least a factor X higher" than the glucosyl transferase activity for a substrate B. This means that, if the k_{cat}/K_{M} values (i.e. the specificity constants) of said glycosyl transferase for substrate A and B are measured under appropriate conditions and the k_{cat}/K_{M} value obtained for the glycosyl transferase with substrate A is divided by the k_{cat}/K_{M} value obtained for the glycosyl transferase with substrate B, the resulting value is X or greater than X.

Similarly, the present application may indicate that a certain glycosyl transferase G "has a glucosyl transferase activity" for a certain substrate A that is "by at least a factor X higher" than the glucosyl transferase activity of another glycosyl transferase H for substrate A. This means that, if the k_{ca}t/K_{M} values of glycosyl transferase G and of glycosyl transferase H for substrate A are measured under appropriate conditions and the k_{cat}/K_{M} value obtained for glycosyl transferase G with substrate A is divided by the k_{cat}/K_{M} value obtained for glycosyl transferase H with substrate A, the resulting value is X or greater than X.

The k_{cat}/K_{M} value can be determined by standard procedures known to the person of skilled in the art. Preferably, recombinant glycosyl transferases are used for determining the k_{cat}/K_{M} values.

Preferably, the following procedure is used:
The kinetic data are determined with increasing concentrations of the substrates from 1 µM to 500 µM and a fixed concentration of sugar precursor (for example an UDP-glucose concentration of 108 µM (100 µM unlabeled UDP-glucose and 8 µM UDP-[¹⁴C] glucose), 833 µM (825 µM unlabeled UDP-glucose and 8 µM UDP-[¹⁴C] glucose) or 512.5 µM (500 µM unlabeled UDP-glucose and 12,5 µM UDP-[¹⁴C] glucose)). The total volume is 40 µL and 0.2 µg, 0.5 µg or 5 µg of purified protein is used. The measurements are performed under the following conditions: The assays are carried out at 30 °C for 1.5 h, 30 min or 10 min using a Tris-HCl buffer (100 mM, 10 mM 2-mercaptoethanol, pH 8.5 or pH 7.5). The amount of the purified enzyme and the incubation time can be adapted depending on the counting sensibility. The reaction is stopped by adding 1 µL 24% trichloroacetic acid and glucosides are extracted with 100 µL ethyl acetate. Radioactivity is determined by LSC.

To determine the kinetic data of a sugar precursor (e.g. UDP-glucose), the value of the substrate used (e.g. geraniol) is fixed (1.25 mM or 0.1 mM) and radiolabeled sugar precursor (e.g. UDP-[¹⁴C] glucose) is mixed with non-radiolabeled sugar precursor (in the example UDP-glucose) to obtain concentrations ranging from 5 µM to 100 µM or 25 µM to 500 µM. The K_{M}- and vₘₐₓ-values are calculated from Lineweaver-Burk plots, Hanes-Woolf plots and non-linear fitting of the experimental data.

At some instances, the present application indicates that a certain glycosyl transferase can be "expressed more efficiently as a recombinant protein in *E.coli* cells" than another glycosyl transferase. The efficiency of recombinant protein expression in *E.coli* can be compared as follows: Recombinant expression of the different glycosyl transferases is carried out in *E.coli* cells by standard methods known to the skilled person, preferably according to the methods described in Example 1 below. Whole-cell extracts from the *E.coli* cells are prepared and proteins in the whole-cell extract are compared after gel-electrophoresis and visualization by coomassie-staining.

As "host cell" transfected with the nucleic acid molecule as described above, the cell of a prokaryotic or eukaryotic organism may be used. As the prokaryotic organism, bacteria, for example, commonly used hosts such as bacteria belonging to genus Escherichia such as Escherichia coli can be used. Alternatively, a cell of a lower eukaryotic organism such as eukaryotic microorganisms including, for example, yeast (e.g. Saccharomyces cerevisiae) or fungi like Aspergillus oryzae and Aspergillus niger can be used. Animal cells or plant cells also can be used as a host. Examples of animal cells that can be used include cell lines of mouse, hamster, monkey, human, etc., as well as insect cells such as silkworm cells and adult silkworm per se.

Construction of a vector may be performed using a restriction enzyme, ligase etc. according to a standard method known in the art. An "expression vector" is a vector that allows expression of a protein encoded by the DNA sequence of the vector in a target cell. The transformation of a host with an (expression) vector can be performed according to standard methods.

At some instances, the present application refers to a host cell being "transfected". This refers to a situation where foreign DNA is introduced into a cell. A transfected host cell may be "stably transfected". This refers to the introduction and integration of foreign DNA into the genome of the transfected cell. Alternatively, a transfected host cell may be "transiently transfected". This refers to the introduction of foreign DNA into a cell where the foreign DNA fails to integrate into the genome of the transfected cell.

As used herein, the term "transgenic plant" refers to a plant that has a heterologous gene integrated into its genome and that transmits said heterologous gene to its progeny. A "heterologous gene" is a gene that is not in its natural environment. For example, a heterologous gene includes a gene from one species introduced into another species. In some embodiments, a heterologous gene also includes a gene native to an organism that has been altered in some way (e.g., mutated, added in multiple copies, or linked to non-native regulatory sequences). Heterologous genes are distinguished from endogenous genes in that the heterologous gene sequences are typically joined to DNA sequences that are not found naturally associated with the gene sequences in the chromosome or are associated with portions of the chromosome not found in nature (e.g., genes expressed in loci where the gene is not normally expressed).

A "protein having glycosyl transferase activity" is a protein that is capable of catalyzing a glycosylation reaction in which the sugar group of a sugar donor is transferred to an acceptor molecule. A protein having glycosyl transferase activity can be obtained by culturing, cultivating or growing a host cell or organism that expresses such a protein (for example a host cell transformed with a vector as described in the above embodiments), and then by recovering and/or purifying the protein from the host cell, host organism or culture medium according to standard methods, such as filtration, centrifugation, cell disruption, gel filtration chromatography, ion exchange chromatography and the like. For example, the methods described in Example 1 of the present application can be used.

A "recombinantly expressed" glycosyl transferase is a glycosyl transferase protein that has been expressed from a recombinant DNA molecule, i.e. from a DNA molecule formed by laboratory methods of genetic engineering (such as molecular cloning) to bring together genetic material from multiple sources, creating a DNA sequence that would not be found naturally in a biological organism. Typically, a recombinantly expressed glycosyl transferase is expressed by heterologous expression (i.e. in a host organism which is different from the organism from which said glycosyl transferase is originally derived), such as by expression in e.g. *E. coli, Saccharomyces cerevisiae, Pichia pastoris* or insect cells, preferably in *E. coli.* Preferably, said recombinantly expressed glycosyl transferase is expressed by heterologous expression. Preferably, said recombinantly expressed glycosyl transferase is isolated after expression from other proteins of the host organism by methods of protein purification.

The present inventors have surprisingly found that VvGT14 and VvGT15 have glucosyl transferase activities (k_{cat}/K_{M}) for the substrates geraniol, nerol and citronellol that are higher by a factor of 2,6 to 44 compared to known terpene glycosyl transferases, such as UGT85B1 of *Sorghum bicolor* (see Table 5). Moreover, the present inventors have surprisingly found that the glycosyl transferases VvGT14 and VvGT15 are expressed more efficiently than other known terpene glycosyl transferases as recombinant proteins in *E.coli* cells or other host cells. Moreover, the present inventors have surprisingly found that the glycosyl transferase VvGT14 is capable of catalyzing glucosylation of furaneol, whereas plant glycosyl transferases that are capable of catalyzing glucosylation of furaneol are otherwise not known.

In the following, reference is made to the figures:
All methods mentioned in the figure descriptions below were carried out as described in detail in the examples.

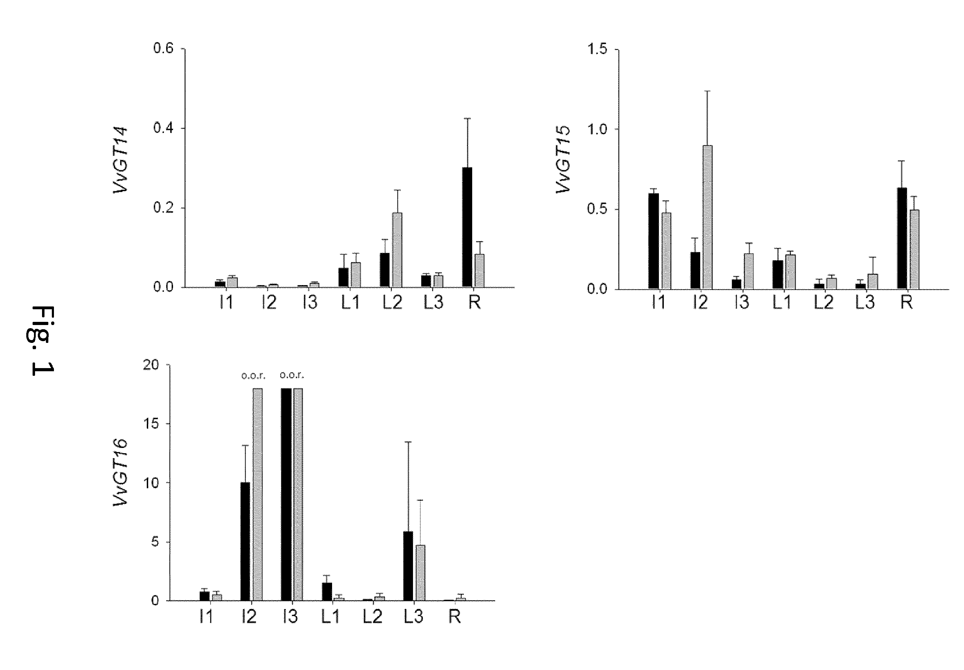

Figure 1 shows experimental data from a gene expression analysis of *VvGTs* by GeXP (Genome Lab GeXP Genetic Analysis System to quantify transcript abundance, see below, Example 1, section "Transcription analysis") in non-berry tissues.
The relative expression was quantified in Gewurztraminer 11-18 Gm (black bars) and White Riesling 239-34 Gm (grey bars). Sampled tissues: Inflorescences four weeks (I1) and two weeks (I2) before flowering and at full bloom (I3), leaves at the age of approximately one week (L1), three weeks (L2) and five weeks (L3) and roots (R). The mean values +SD of three independent experiments are shown, o.o.r. out of range.

Figure 2 shows experimental data from a gene expression analysis of *VvGTs* by GeXP. Different stages of berry development are given as weeks post flowering. Expression was determined in berry skins (exocarp) of five different varieties and clones. Mean values ± SD of three independent experiments are shown, o.o.r. out of range.

Figure 3 shows data on the relative specific activity (%) of VvGT14a (A), VvGT15a-c (B), and VvGT16 (C) protein from *Vitis vinifera* towards putative substrates as determined by radiochemical analysis with UDP-[¹⁴C]glucose (see below, Example 1).
The relative activities refer to the highest level of extractable radioactivity which was measured for the conversion of geraniol (100 %) in case of VvGT14a and VvGT15a-c (order of the columns 15c, 15b, 15a) and benzyl alcohol (100 %) in the case of VvGT16. Data for two biological and two technical replicates are shown. Black and white bars represent monoterpenoids and non-monoterpenoids, respectively. (D): Chemical structures of geraniol, nerol, 3*S*-citronellol, α-terpineol and 3S-linalool.

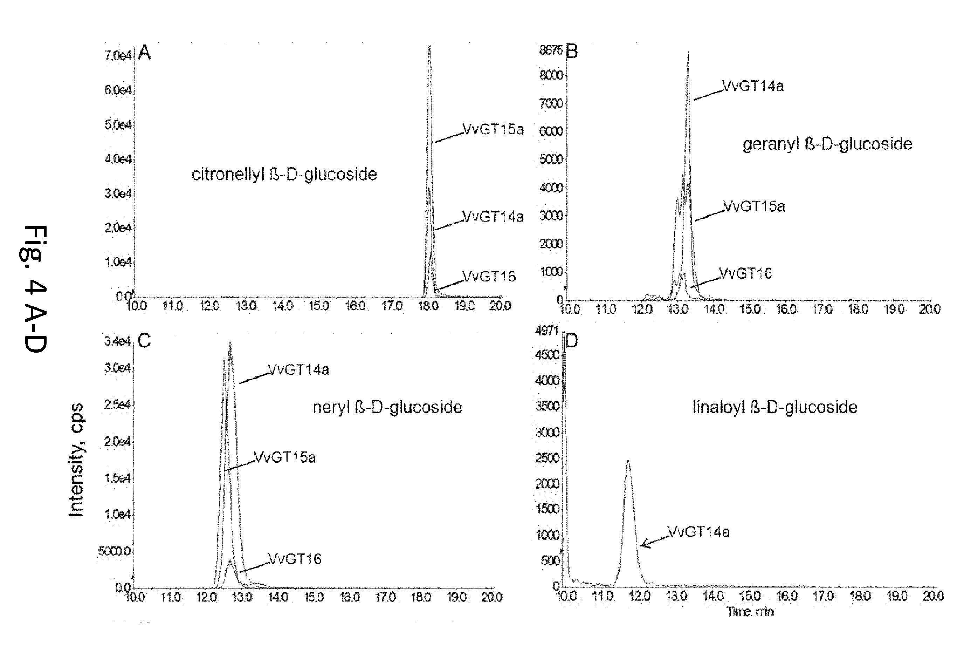

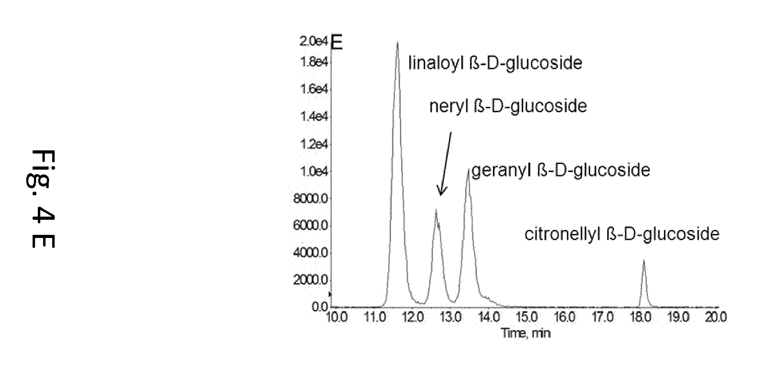

Figure 4 shows the detection of monoterpenyl ß-D-glucosides as products of VvGT14a, VvGT15a and VvGT16 by LC-MS.
LC-MS analysis of citronellyl ß-D-glucoside (A), geranyl ß-D-glucoside (B), neryl ß-D-glucoside (C), linaloyl ß-D-glucoside (D) formed by VvGT14a, VvGT15a and VvGT16 and a mixture of synthesized monoterpenyl ß-D-glucosides (E); chromatograms display an overlay of single product measurements, traces show the total ion current of the characteristic transitions (refer to Example 1), Gaussian smoothing was partly applied.

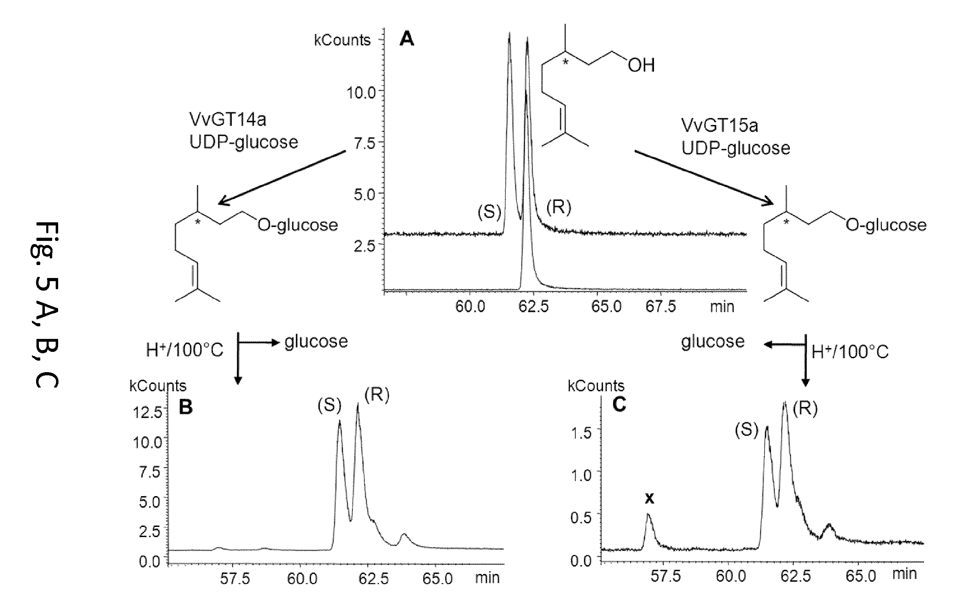

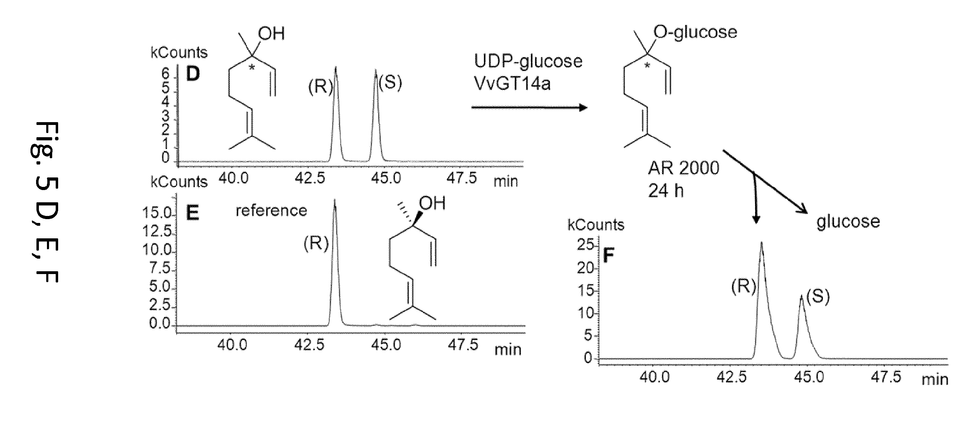

Figure 5 shows experimental data on the enantioselectivity of VvGT14a and VvGT15a as determined by chiral phase SPME-GC-MS analysis of citronellol and linalool.
A) Racemic mixture of *R,S*-citronellol was used as substrate for VvGT14a and VvGT15a, enantiomerically pure *R*-citronellol was used as reference.
B, C) racemic citronellol is released by acid catalyzed hydrolysis of citronellyl-ß-D-glucoside formed by VvGT14a and VvGT15a. Signals labeled with "x" are hydrolysis by-products. Chromatograms are shown in SIM mode by using the characteristic ion traces *m*/*z* 69, 81 and 123 for citronellol.
D) Racemic mixture of *R,S*-linalool was used as substrate for VvGT14a.
E) Enantiomerically pure *R*- linalool was used as reference material.
F) A slight preference for the *R*-linalool is revealed after enzymatic hydrolysis with AR 2000. Chromatograms are shown in SIM mode (*m*/*z* 71, 93).

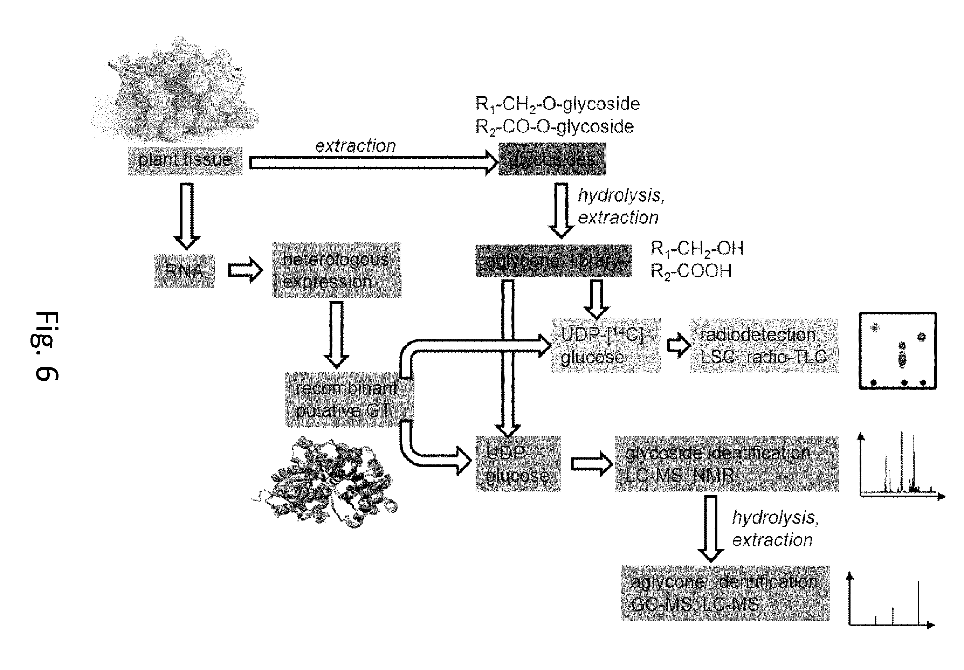

Figure 6 shows a flow chart of the formation and use of a physiologic aglycone library. An aglycone library is prepared from a tissue that shows high *UGT* transcript levels. The library is screened with recombinant UGT enzymes and various labeled and unlabeled UDP-sugars. Formation of products can be rapidly quantified by LSC and confirmed by TLC.

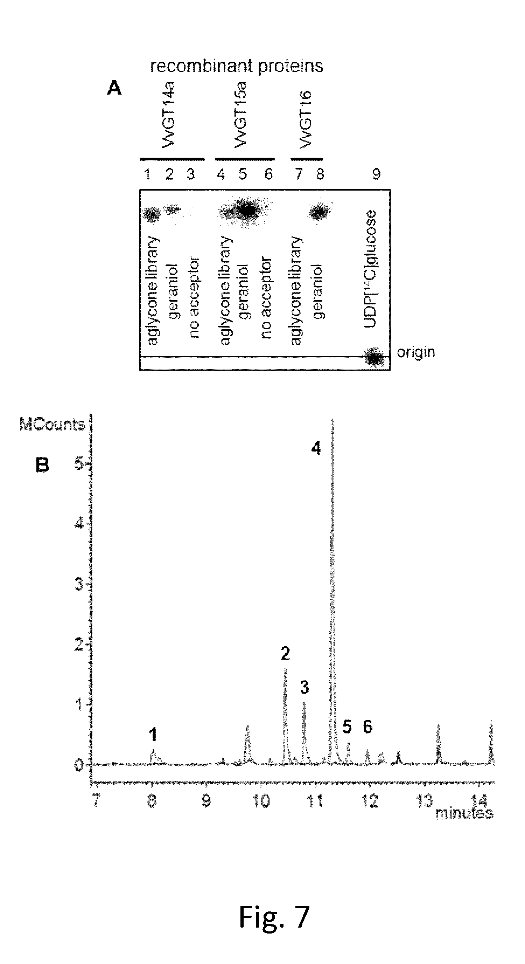

Figure 7 shows experimental data obtained from functional screening of VvGT14a, 15a and 16 by radio-TLC and GC-MS analysis.
Radio-TLC analysis (A) of products formed by VvGT14a (1,2,3), VvGT15a (4,5,6) and VvGT16 (7,8,9) after incubation with the aglycone library obtained from grape berries of Gewurztraminer 11-18 Gm (1,4,7); Positive control: geraniol (2,5,8); negative control: no acceptor molecule (3,6); UDP [¹⁴C]-glucose (9; approximately: 3000 dpm). The plates were analyzed by digital autoradiograph. The products formed from citronellol, geraniol and nerol were verified by LC-MS analysis. GC-MS analysis (B, total ion chromatogram) of volatiles that were enzymatically released from glucosides which were formed by incubation of an aglycone library obtained from grape berries of Gewurztraminer 11-18 Gm with UDP-glucose and VvGT14 (gray) or heat-inactivated VvGT14a (black) as control 1: linalool; 2: citronellol; 3: nerol; 4: geraniol; 5: benzyl alcohol; 6: phenylethanol.

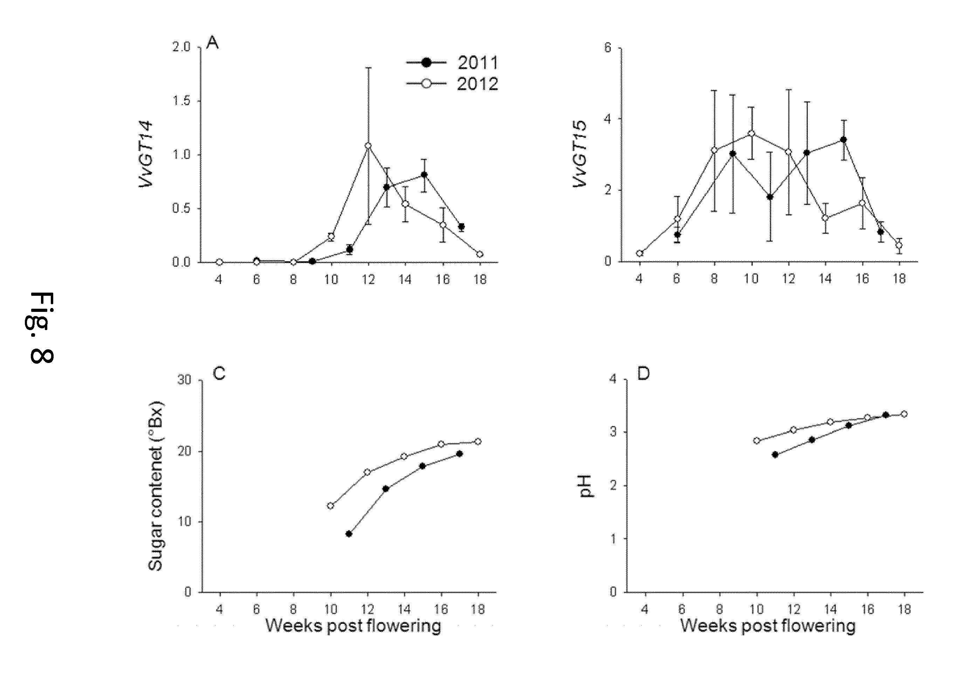

Figure 8 shows experimental data from a gene expression analysis of *VvGTs* by GeXP. Gene expression of *VvGTs* by GeXP (A) was analyzed in berry skins (exocarp) of Muscat FR 90 from two consecutive years (2011, 2012). Mean values ± SD of three independent experiments are shown. The ripening related parameters sugar content (C) and pH (D) were quantified after veraison in must obtained from 100 berries of Muscat FR 90 by refractometer and pH meter.

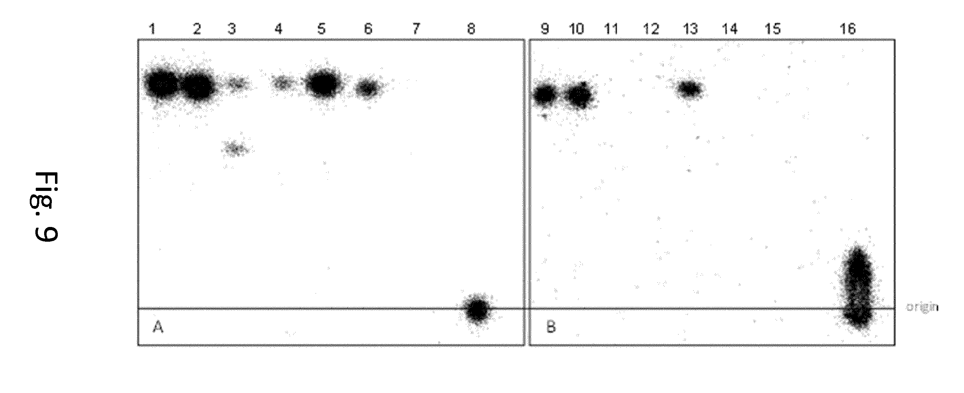

Figure 9 shows experimental data from radio-TLC analysis of products formed by (A) VvGT14a and (B) VvGT16 from different monoterpenes. Citronellol (1,9), geraniol (2,10), 8-hydroxylinalool (3,11), linalool (4,12), nerol (5,13), terpineol (6,14), no substrate (7,15), UDP [¹⁴C]-glucose (8,16, approximately: 3000 dpm). The plates were analyzed by digital autoradiograph. The putative glucosidic products formed from citronellol, geraniol and nerol were verified by LC-MS analysis.

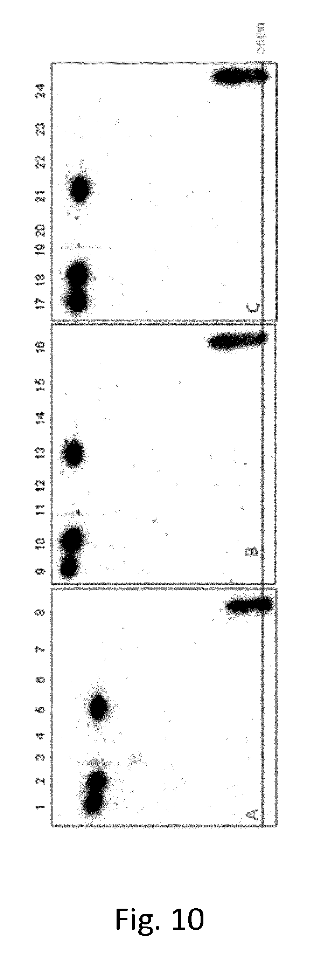

Figure 10 shows experimental data from radio-TLC analysis of products formed by (A) VvGT15a, (B) VvGT15b and (C) VvGT15c from different monoterpenes. Citronellol (1,9,17), geraniol (2,10,18), 8-hydroxylinalool (3,11,19), linalool (4,12,20), nerol (5,13,21), terpineol (6,14,22), no substrate (7,15,23), UDP [¹⁴C]-glucose (8,16,24; approximately: 3000 dpm). The plates were analyzed by digital autoradiograph. The putative glucosidic products formed from citronellol, geraniol and nerol were verified by LC-MS analysis.

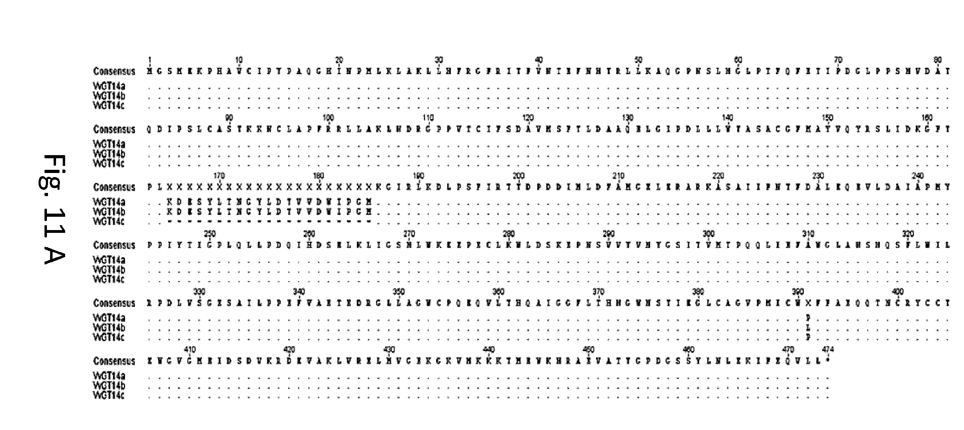

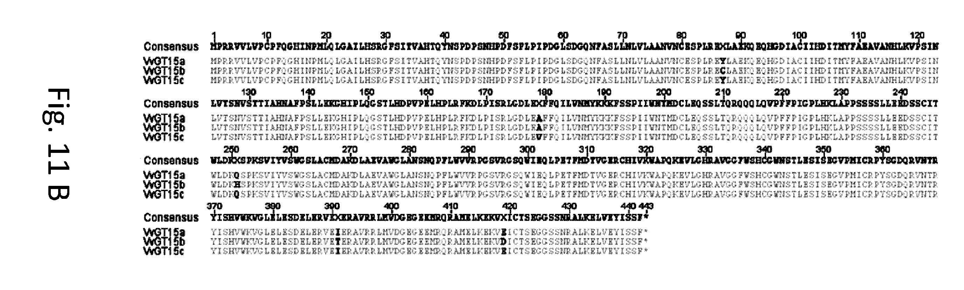

Figure 11 shows a multiple protein sequence alignments of the three variants of VvGT14 and of VvGT15, respectively. (A) Multiple protein sequence alignment of the three variants of VvGT14. The alignment was performed using MUSCLE alignment in Geneious Pro 5.5.6 software (Biomatters). VvGT14a was catalytically active while VvGT14b and VvGT14c did not show any enzymatic activity. (B) Multiple protein sequence alignment of the three variants of VvGT15.

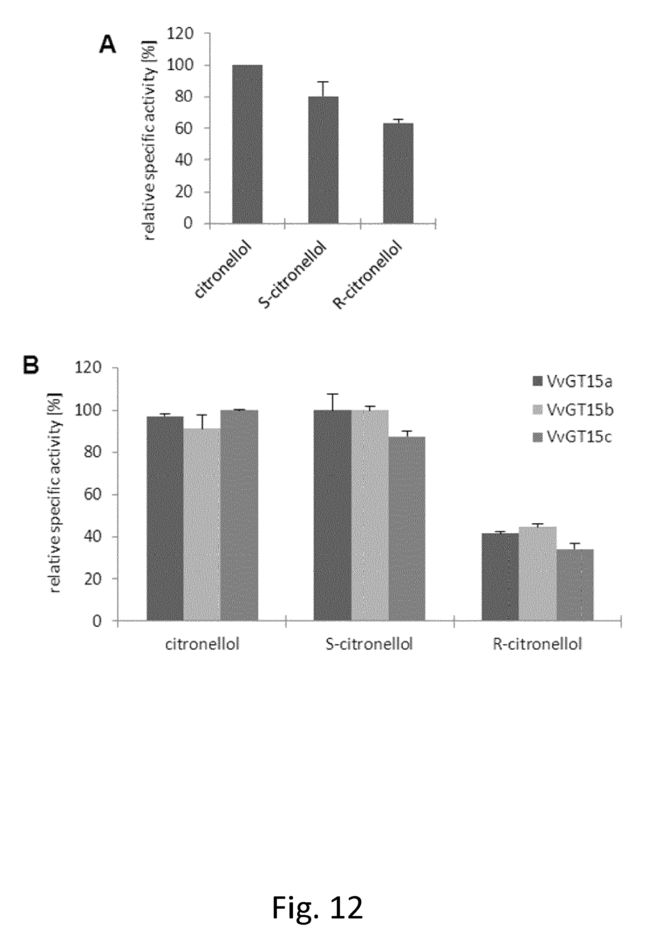

Figure 12 shows experimental data on the enantiomeric discrimination of VvGT enzymes. Relative specific activity (%) of VvGT 14a protein from *Vitis vinifera* towards citronellol and enantiomerically pure R- and S-citronellol (A). The relative activities refer to the highest level of extractable radioactivity which was measured for VvGT14a with the racemic citronellol (100 %). Relative specific activity (%) of VvGT15a-c proteins from *Vitis vinifera* towards racemic citronellol and enantiomerically pure R- and S-citronellol (B). The relative activities refer to the highest level of extractable radioactivity (100 %) which was measured for each protein with racemic citronellol (VvGT15c) or S-citronellol (VvGT15a and VvGT15b).

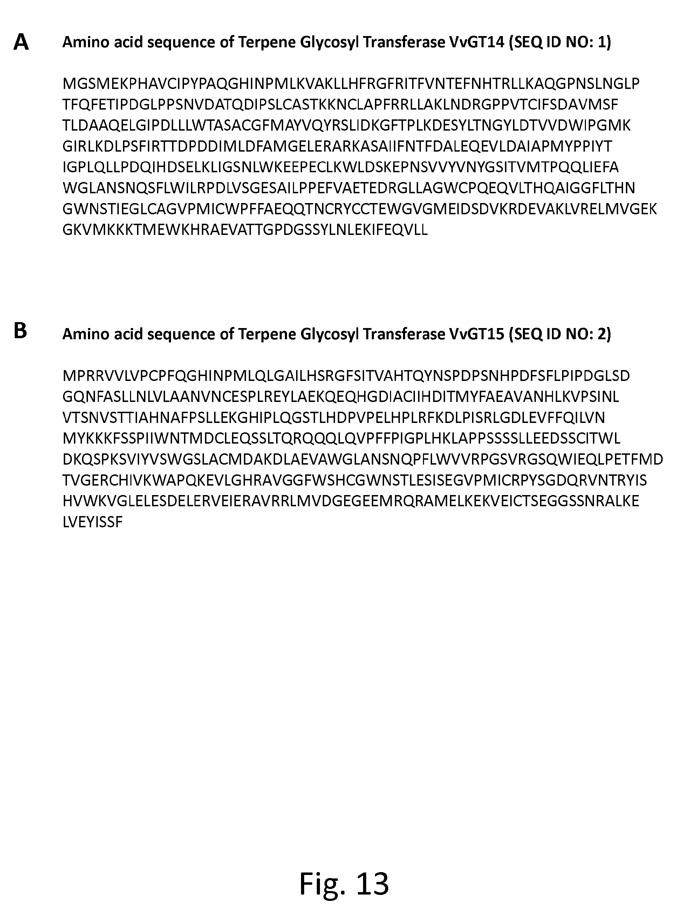

Figure 13 shows the amino acid sequences of terpene glycosyl transferases VvGT14 and VvGT15. (A): Amino acid sequence of terpene glycosyl transferase VvGT14 (SEQ ID NO: 1). (B): Amino acid sequence of terpene glycosyl transferase VvGT15 (SEQ ID NO: 2).

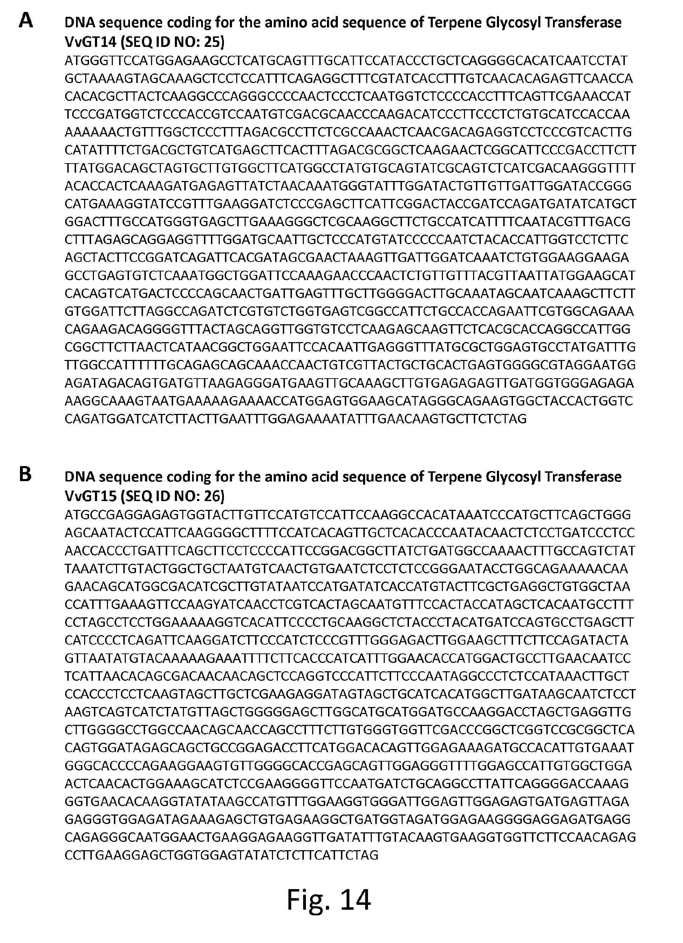

Figure 14 shows DNA sequences coding for terpene glycosyl transferases VvGT14 and VvGT15, respectively. (A): A DNA sequence coding for the amino acid sequence of terpene glycosyl transferase VvGT14 (SEQ ID NO: 3). (B): A DNA sequence coding for the amino acid sequence of terpene glycosyl transferase VvGT15 (SEQ ID NO: 4).

In the following, reference is made to the examples, which are given to illustrate, not to limit the present invention.

### Examples

### Example 1

### Plant Material

*Vitis vinifera* grapevines of cultivars Gewurztraminer 11-18 Gm, Gewurztraminer FR 46-107, White Riesling 239-34 Gm, White Riesling 24-196 Gm and Muscat a petit grains blancs FR 90 were grown during vintages 2011 and 2012. Grape berries, leaves, inflorescences and roots were collected. Sampling was conducted for a total of six dates between 6 and 17 weeks after bloom including berries from pea-size to harvest ripeness. Muscat a petit grains blancs FR 90 was additionally sampled every two weeks from week 4 to week 18 after bloom.

After veraison (the onset of ripening) 100 berries were collected for the determination of ripening related parameters like sugar content. For terpenoid analysis 250 g of berries were stored at -20 °C, while three replicates consisting of ten berries were peeled and skins immediately frozen in liquid nitrogen for subsequent RNA extraction. Roots were obtained from scions of White Riesling 239-34 Gm and Gewurztraminer 11-18 Gm grown in the greenhouse. Leaves were sampled from the same cultivars at the approximate age of one, three and five weeks. In addition, inflorescences four and two weeks before flowering and at full bloom were collected. Samples were stored at -20°C until work-up.

### Chemicals

UDP-[¹⁴C]glucose (300 mCi/mmol, 0.1 mCi/mL) was obtained from American Radiolabelled Compounds (St Louis, MO, USA). (*R,S*)-3,7-dimethyl-1,6-octadien-3-ol (linalool) and (*E*)-3,7-dimethyl-2,6-octadien-1-ol (geraniol) were obtained from Roth (Karlsruhe, Germany). (*R,S*)-3,7-dimethyl-6-octen-1-ol (citronellol) and pure (*R*)-(+)-ß-citronellol were purchased from Sigma Aldrich (Steinheim, Germany). (*Z*)-3,7-dimethyl-2,6-octadien-1-ol (nerol) was purchased from Alfa Aesar (Karlsruhe, Germany). (*R,S*)-3,7-dimethyl-6-octenyl ß-D-glucopyranoside (citronellyl ß-D-glucoside), (*Z*)-3,7-dimethyl-2,6-octadienyl ß-D-glucopyranoside (neryl ß-D-glucoside) and (*E*)-3,7-dimethyl-2,6-octadienyl ß-D-glucopyranoside (geranyl ß-D-glucoside) were synthesized according to the Koenigs-Knorr-procedure, (*R,S*)-3,7-dimethyl-1,6-octadienyl ß-D-glucopyranoside (linaloyl ß-D-glucoside), according to a modified Koenigs-Knorr-procedure.

### Sample preparation for metabolite analysis

Grapes berries were peeled and 10 g (fresh weight) of the grape skins were taken for one analysis. In case of root, leaf and inflorescence, 4 g plant material was taken per analysis. The material was frozen in liquid nitrogen, ground and extracted with a mixture of phosphate buffer (0.1 M, pH 7) and 13% ethanol for 24 h under nitrogen with exclusion of light (Jesus Ibarz et al., 2006). 2-Octanol was used as internal standard for the determination of free monoterpenes. For the determination of monoterpenyl ß-D-glucosides, stable isotope dilution analysis (SIDA) was applied, using [²H₂]-citronellyl ß-D-glucoside as a labeled, internal standard. The concentration of the internal standards was adapted for the variety, the tissue and the ripening stage of the plant material. 2-Octanol was added in a range of 0.3 to 6.8 mg/kg plant material, [²H₂]-citronellyl-ß-D-glucoside in a range of 0.1 to 3.5 mg/kg plant material.

To purify the sample, Carrez reagents (Merck Millipore, Darmstadt, Germany) were added (1 mL each) and the sample was then centrifuged at 14500 rpm for 20 min at 5 °C. The supernatant was taken for subsequent solid phase extraction (SPE) to isolate and separate free monoterpenes from glycosidically bound monoterpenes. Therefore, a 200 mg Lichrolut EN column (Merck, Darmstadt, Germany) was conditioned as described (Piñeiro et al., 2004). Free monoterpenols were eluted with dichloromethane and glycosidically bound monoterpenols with methanol.

For GC-MS detection, the dichloromethane fractions were dried with Na₂SO₄, concentrated using nitrogen to 200 µL and analyzed. For LC-MS detection, the methanolic fractions were concentrated under reduced pressure and the residues were dissolved in water/acetonitrile (7/3; v:v). The samples were analyzed by LC-MS.

### Nucleic acid extraction

For total RNA extraction plant material was ground to a fine powder in liquid nitrogen using mortar and pestle. One g of the powder was used for RNA extraction with the CTAB (cetyltrimethylammonium bromide) method following an established protocol (Zeng and Yang, 2002, adapted by Reid et al. 2006) to meet the requirements of different grape tissues. Remaining genomic DNA was digested by DNase I and cleaned up with the High Pure RNA Isolation kit (Roche, Mannheim, Germany).

### Transcription analysis

Transcription analysis was performed using the Genome Lab GeXP Genetic Analysis System (Beckman Coulter, Krefeld, Germany), a multiplex quantitative gene expression analysis system. The gene expression patterns of the *VvGT* genes *VvGT14, VvGT15* and *VvGT16* and five reference genes (*VvActin, VvAP47, VvPP2A, VvSAND,* and *VvTIP41*) were analyzed simultaneously from one sample of total RNA.

Reverse transcription was carried out with the GenomeLab^{™} GeXP Start kit (Beckman Coulter) following the manufacturer's instructions. As an internal control gene KAN^{r} RNA was co-reverse transcribed and subsequently amplified together with the reference genes and genes of interest. The gene specific primers for reverse transcription are chimeric, providing a 19 nt universal tag for the binding of universal reverse primers in the subsequent PCR reaction. The final concentration of the primers ranged from 0.1 nM to 100 nM to compensate for the different transcription levels of the analyzed genes (Table 3). Multiplex PCR reactions were conducted with Thermo-Start DNA Polymerase (Thermo Fisher Scientific, Dreieich, Germany). Each reaction contained 9.3 µL of reverse transcription products as template and 10.7 µL of a PCR reaction mix including gene specific forward primers providing an 18 nt universal tag (Table 3). The universal forward primer is labeled with a fluorescent dye for detection during subsequent capillary electrophoresis. Primer pairs were designed to yield PCR products ranging from 119 bp to 374 bp and differing in size by at least 8 bp. Of each PCR product, 4 µL were separated by capillary electrophoresis using the GenomeLab Genetic Analysis System (Beckman Coulter).

Individual standard curves for each gene in the multiplex were performed with serial two-fold dilutions ranging from 3.91 ng to 500 ng of an RNA mixture from all samples. Raw data were analyzed using the fragment analysis tool. The fragment data of the standard curves and samples were then normalized to the peak area of KAN^{r} RNA with the express analysis tool. Subsequently, the relative signal level of each sample replicate was interpolated from the standard curve. The data was further normalized to the geometric mean of the five reference genes with quant tool. All software for GeXP data analysis was purchased from Beckman Coulter.

### Cloning of VvGT14, VvGT715 and VvGT16 DNA sequences from Vitis vinifera

The reference genome of *Vitis vinifera* sequenced genome PN40024 was used to design gene specific primers in the untranslated regions of the three putative VvGT genes, VvGT 14, VvGT15, and VvGT16 using the tool Primer-BLAST. Primers (Table 4) were purchased from Eurofins MWG Operon (Ebersberg, Germany). The cDNA-synthesis was performed with the SuperScript® III First-Strand Synthesis SuperMix (Life Technologies, Darmstadt, Germany) following the manufacturer's instructions. The template for cDNA-synthesis was total RNA, extracted from grape exocarp. The cDNA was used as template in the following PCR-reaction. PCR was performed with Phusion DNA Polymerase (Thermo Fisher Scientific, Dreieich, Germany) using high-fidelity (HF)-buffer and the following thermal cycling conditions: 98 °C for 30 s followed by 32 cycles consisting of 98 °C for 5 s, 60 °C for 5 s and 72 °C for 30 s and a final elongation step of 72 °C for 1 min.

PCR products were gel purified with the Wizard SV Gel and PCR Clean-Up System (Promega, Madison, USA). A-tailing of purified PCR-products was performed with *Taq* DNA Polymerase (Thermo Fisher Scientific). A-tailed PCR-products were ligated into pGEM-T Easy vector (Promega, Madison, USA) and cloned in One Shot TOP10 Chemically Competent *E.Coli* (Life Technologies, Darmstadt, Germany). Plasmids were isolated with the PureYield Plasmid Miniprep System (Promega, Madison, USA) and sequenced with the vector specific primers M13 uni (-21) and M13 rev (-29) on an ABI 3730 capillary sequencer (StarSEQ, Mainz, Germany). Raw data was edited with the FinchTV software (Geospiza, Seattle, USA). Sequences were assembled with SeqMan and aligned with MegAlign (DNASTAR, Madison, USA).

### Construction of expression plasmids

The full-length ORFs of the *VvGT* sequences were subcloned into the pGEM-Teasy vector (Promega, Madison, WI, USA). All genes were amplified with primer introducing BamHI und NotI restriction sites. Subsequently, the genes were cloned *in frame* with the N-terminal GST-tag into the pGEX-4T-1 expression vector (Amersham Bioscience, Freiburg, Germany). The gene identity was confirmed by sequencing (Eurofins MWG Operon, Ebersberg, Germany).

### Heterologous protein expression

Recombinant protein was expressed in *E. coli* BL21 (DE3) pLysS (Novagen, Schwalbach, Germany). Pre-cultures were grown overnight at 37 °C in Luria Bertani medium containing 100 µg/mL ampicillin and 23 µg/mL chloramphenicol. The next day, 1 L Luria Bertani solution containing the appropriate antibiotics was inoculated with 50 mL of the pre-culture. The culture was grown at 37 °C at 160 rpm until OD₆₀₀ reached 0.6-0.8. After cooling the culture to 16 °C, 1 mM IPTG was added to induce protein expression. Cultures were incubated over night at 16-18 °C at 160 rpm. Cells were harvested by centrifugation and stored at -80 °C. Negative controls were carried out with *E. coli* BL21 (DE3) pLysS cells containing the empty expression vector pGEX-4T-1.

### Cell lysis and purification

Recombinant GST-fusion proteins were purified by GST Bind resin (Novagen) following the manufacturer's instructions. Briefly, the cells were re-suspended in the binding buffer containing 10 mM 2-mercaptoethanol. Cells were disrupted by sonication. The crude protein extract was incubated for 2 h with the GST Bind resin to bind GST fusion protein. The recombinant protein was eluted with GST elution buffer containing reduced glutathione and quantified by Bradford solution (Sigma-Aldrich, Steinheim, Germany). The presence of the expressed proteins was confirmed by SDS-PAGE and Western Blot using anti-GST antibody.

### Activity assay and kinetics

In the initial screening, each reaction mixture (200 µL in total) contained Tris-HCl buffer (100 mM, pH 8, 10 mM 2-mercaptoethanol), 37 pmol UDP-[¹⁴C]glucose (0.01 µCi, Biotrend, Köln, Germany), substrate (50 µL of a 1 mg/mL stock solution) and purified protein (0.5-0.8 µg/µL). The reaction mixture was incubated at 30 °C for 18.5 hours. The assays were stopped by adding 1 µL 24% trichloroacetic acid and extracted with 500 µL water-saturated 1-butanol. The organic phase was mixed with 2 mL Pro Flow P+ cocktail (Meridian Biotechnologies Ltd., Epsom, UK) and radioactivity was determined by liquid scintillation counting (LSC, Tri-Carb 2800TR, Perkin Elmer, Waltham MA, USA). Additionally, negative controls without substrate were performed. After determining the optimal conditions for the best substrate of each gene, the substrate screening was repeated under these conditions.

The kinetic data were determined with increasing concentrations of the substrates (VvGT14a: citronellol, geraniol, 8-hydroxylinalool, linalool, nerol and terpineol; VvGT15a-c: *S-*citronellol, geraniol, 8-hydroxylinalool and nerol; VvGT16: citronellol, geraniol and nerol) from 1 µM to 100 µM (VvGT14a and VvGT15a-c) or 50 µM to 500µM (VvGT16) and a fixed UDP-glucose concentration of 108 µM (100 µM unlabeled UDP-glucose and 8 µM UDP-[¹⁴C] glucose; VvGT14a), 833 µM (825 µM unlabeled UDP-glucose and 8 µM UDP-[¹⁴C] glucose; VvGT15a-c) or 512.5 µM (500 µM unlabeled UDP-glucose and 12,5 µM UDP-[¹⁴C] glucose; VvGT16). The total volume was 40 µL and 0.2 µg (VvGT14a), 0.5 µg (VvGT15a-c) or 5 µg (VvGT16) of purified protein. The measurements were performed under the following conditions. The assays were carried out at 30 °C for 1.5 h using a Tris-HCl buffer (100 mM, 10 mM 2-mercaptoethanol, pH 8.5 for VvGT14a and VvGT16). The assay of VvGT15a-c was performed at 30 °C using a Tris-HCl buffer (100 mM, 10 mM 2-mercaptoethanol, pH 7.5) and 10 min (VvGT15c) or 30 min (VvGT15a-b) of incubation for the best substrates. The amount of the purified enzyme and the incubation time were adapted depending on the counting sensibility. The reaction was stopped by adding 1 µL 24% trichloroacetic acid and glucosides were extracted with 100 µL ethyl acetate. Radioactivity was determined by LSC.

To determine the kinetic data of UDP-glucose, the value of geraniol was fixed (1.25 mM for VvGT15a-c and VvGT16; 0.1 mM for VvGT14a) and UDP-[¹⁴C] glucose was mixed with non-radiolabeled UDP-glucose to obtain concentrations ranging from 5 µM to 100 µM (VvGT14a and VvGT15a-c) or 25 µM to 500 µM (VvGT16). The K_{M}- and vₘₐₓ-values were calculated from Lineweaver-Burk plots, Hanes-Woolf plots and non-linear fitting of the experimental data.

### Preparation of aglycone libraries

For the preparation of aglycone extracts 50 g (fresh weight) grape skins or 4 g (fresh weight) inflorescences of Gewurztraminer Gm 11-18 were ground to a fine powder in liquid nitrogen. The isolation of glycosides was carried out as described above (see metabolite analysis). The methanolic fraction, which was obtained after SPE, was enzymatically hydrolysed. Therefore, the dried sample was dissolved in citric-acid buffer (0.1 M, pH 4), 50 mg AR 2000 (an enzyme preparation with glucosidase activity prepared from *Aspergillus niger;* DSM Food Specialties Beverage Ingredients, Delft, Netherlands) was added and incubated for 24 h with exclusion of light. The liberated aglycones were extracted by 20 mL methyl-*tert*-butyl ether and the organic phase was reduced to 1000µL using a gentle stream of nitrogen.

### Activity based profiling using a physiologic aglycone library

Aliquots of this aglycone extract were incubated with UDP-glucose and various VvGT-enzymes. Optimum conditions at 30 °C for 24 hours were applied. Each solution contained 100 µL purified enzyme, 100-150 µL Tris-HCl buffer (100 mM, pH7.5 or 8.5, 10 mM 2-mercaptoethanol), 37 pmol UDP-[¹⁴C]glucose (0.01 µCi) and 50-100 µL extract (dissolved in methyl-tert-butylether). The buffer was mixed with the extract and the organic solvent was gently vaporized with nitrogen. The missing volume was adjusted with buffer before the enzyme was added. The reaction was stopped by adding 1 µL 24% trichloroacetic acid and extracted with 500 µL ethyl acetate. After termination of the reaction free aglycones, which were not converted by VvGT14 and VvGT15, were measured via SPME-(Solid Phase Microextraction)-GC-MS. These residual, free aglycones were completely removed by extraction with dichloromethane. Enzymatically formed glucosides were extracted by ethyl acetate, which was removed under nitrogen. The residue was dissolved in methanol and analyzed by LC-MS to detect the generated monoterpenol glucosides. Enzymatic hydrolysis of the glucosides was performed using AR 2000 and 2 mL citric-acid-buffer. After hydrolysis, a 100 µL aliquot was used to detect volatile aglycones via SPME-GC-MS. The remaining solution was extracted with methyl-*tert*-butyl ether and reduced under nitrogen to approximately 200 µL. One µL were measured by GC-MS via liquid injection.

### Enantioselectivity of VvGT14a, VvGT15a-c

To determine the enantioselectivity of VvGT14 and VvGT15 the enantiomeric ratio of glucosidically bound citronellol and linalool was determined by enantioselective GC-MS. Following the incubation, residual citronellol and linalool were completely removed by extraction with dichloromethane. Citronellyl ß-D-glucoside which remained in the aqueous phase was hydrolyzed by HCl (2 mL, 0.1 M, pH 1) for one hour at 100 °C to release citronellol (Skouroumounis and Sefton, 2000). In case of linalyl ß-D-glucoside an enzymatic hydrolysis (AR 2000, citric acid buffer pH 4, 24 h) was applied due to the instability of linalool in acid solutions (Williams et al., 1982). Hydrolysis of a synthetic 1:1 mixture of *R-*and S-linalyl ß-D-glucoside revealed that AR 2000 does not discriminate between the two diastereomeric glucosides. After hydrolysis, citronellol and linalool were analyzed by SPME-GC-MS as described above.

### LC-MS analysis

For LC-MS analysis of monoterpenyl ß-D-glucosides a Shimadzu LC20AD HPLC system coupled to an API 2000 (Applied Biosystems, AB Sciex, Framingham, USA) triple-quadrupol-MS was used. Data acquisition was performed using Analyst software version 1.6.1. (Applied Biosystems, AB Sciex, Framingham, USA). The column (Phenomenex Gemini-NX 5u C18, 250 x 3 mm, Aschaffenburg, Germany) was eluted with a linear gradient starting at water/acetonitrile (7/3; v:v) containing 0.2% ammonia till 12 min to water/acetonitrile (4/6; v:v; 0.2% ammonia) at 18 min. The column temperature was maintained at 40 °C. The mass spectrometer was operated in ESI-MRM negative ion mode. Nitrogen was used as curtain (setting 20), nebulizing and collision gas (collision energy was - 20 eV). Monoterpenyl ß-D-glucosides were identified by the following, characteristic MRM transitions {LinGlc: *m*/*z* 315→161(Glu), 315→113(Glu); NerGlc: *m*/*z* 315→119(Glu), 315→113 (Glu); GerGlc: *m*/*z* 315→119(Glu), 315→113(Glu); CitrGlc: *m*/*z* 317→101(Glu), 317→7161(Glu)} (Cole et al., 1989; Domon and Costello, 1988; Salles et al., 1991).

### GC-MS analysis

GC-MS analysis was performed with a Varian GC-450 coupled to a Varian MS-240 ion-trap employing a Phenomenex Zebron ZB-WAXplus column (30 m x 0.25 mm x 0.25 µm, Aschaffenburg, Germany). Helium flow rate was 1 mL/min. The analysis was carried out in split mode (liquid injections) or splitless mode (SPME measurements) with 220 °C injector temperature. EI (electron impact ionization)-MS spectra were recorded from *m*/*z* 40 to 300 (ionization energy 70 eV; trap temperature 170 °C). The oven temperature program was 60 °C (3 min), 10 °C/min up to 250 °C (5 min). In case of SPME measurements, liberated aglycones were isolated for 10 min at 60°C using a fiber coated with a 85 µm film of polyacrylate (SUPELCO, Bellefonte, USA). After extraction the SPME fiber was desorbed for 10 min at 250°C in the injection port of the GC-MS system and the column oven program was carried out as described above. Enantioselective GC-MS analysis was performed with a Varian GC-450 coupled to a Varian MS-240 ion-trap. The column was a Diacß (heptakis-(2,3-di-O-acetyl-6-O-tert.-butyldimethylsilyl)-ß-cyclodextrin), 26 m x 0.32 mm i.d. with a 0.1 µm film. Helium was used as carrier gas at a flow rate of 1 mL/min, injector temperature was 250 °C with a split ratio of 1/100 (liquid injections), or splitless (SPME measurement). The oven temperature program was 70 °C (3 min), 0.5 °C/min to 130 °C 20 °C/min up to 200 °C (3 min). GC-MS measurements were recorded in full scan mode. Selected ion monitoring (SIM) mode was used for quantification.

### Radio-TLC analysis

Assays containing UDP-[¹⁴C]glucose and aglycone libraries or substrates were performed as described above and subsequently extracted with 500 µL ethyl acetate. The organic solvent was vaporized and the pellet was re-suspended in 10 µL methanol and was applied on Silica Gel 60 F254 plates (Merck, Darmstadt, Germany). The dried plates were developed in a solvent system chloroform:acetic acid:water (50/45/5, v:v:v). Plates were dried and analyzed by digital autoradiograph (digital autoradiograph, EG&G Berthold, Wildbad, Germany).

### Example 2

### Expression analysis - temporal and spatial

All methods mentioned in this example were carried out as described in Example 1.

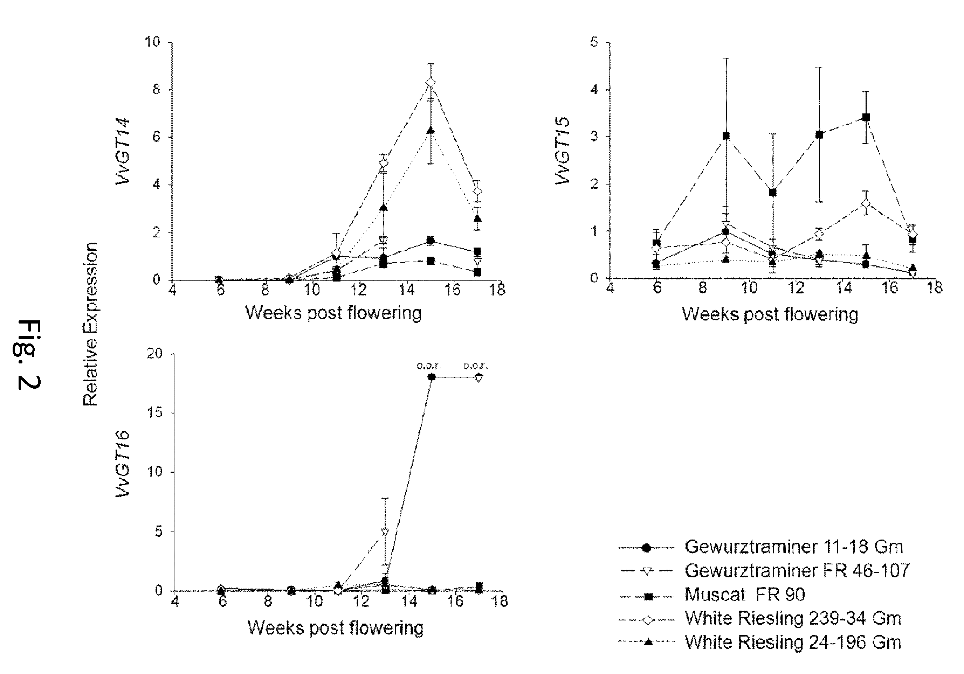

The transcript levels of *VvGT* genes *VvGT14* (designation in *Vitis vinifera* genome project: VIT_18s0001g06060), *VvGT15* (VIT_06s0004g05780) and *VvGT16* (VIT_03s0017g01130) of *Vitis vinifera* cv. Pinot noir were analyzed using GeXP profiling in up to five cultivars in three different tissues (Figure 1) and in grape berry exocarp at different developmental stages (Figure 2). In non-berry tissue (inflorescence, leaf and root) *VvGT14* and *15* showed the lowest relative transcript levels of the genes but displayed a ripening related expression pattern in berry skins similar to *VvGT16.* Significant amount of *VvGT14-16* mRNA was found in berry exocarp at late stages of berry ripening (Figure 1). Notably, their transcript levels differed considerably between varieties. *VvGT14* was expressed primarily in the two surveyed clones of White Riesling, *VvGT15* in Muscat and *VvGT16* in the two clones of Gewurztraminer. Expression profiling was also performed for berry skins of Muscat FR 90 in two subsequent years. In 2011, in comparison to 2012, similar relative transcript levels of *VvGT14-15* were reached, but slightly later (2-3 weeks; Figure 8).

The same is true for the ripening related parameters such as sugar content and pH value. Thus, *VvGT14-16* appear to play an important role in grape berry ripening as their expression levels peak after veraison and they are barely expressed in other tissues, except *VvGT16.*

### Example 3

### Metabolite profiling

All methods mentioned in this example were carried out as described in Example 1.

To correlate the expression profiles of putative *UGTs* with terpenyl glucoside concentration metabolite analysis was performed in five cultivars during grape ripening (Table 1). Solid phase extraction was used to isolate free (non-glycosylated) and glycosylated monoterpenes from grape skins (exocarp) of various grapevine cultivars (Gunata et al., 1988; Mateo and Jiménez, 2000). Since grape skins (exocarp) accumulate the majority of terpene metabolites detected in grape berries they were separated from the flesh and extracted (Wilson et al., 1986). Geraniol, nerol, linalool and citronellol were quantified by GC-MS analysis whereas their non-volatile monoterpenyl glucosides were determined by a stable isotope dilution analysis (SIDA) method using LC-MS. Isotopically labelled internal standards were chemically synthesized.

Grape berries of the *V. vinifera* cultivars differed not only in their amounts of total terpenes, but also in their terpene profiles at different developmental stages (Table 1). Monoterpenols (free and glucosidically bound) were hardly detected (less than 0.25 mg/kg grape skins) in grape exocarp of Gewurztraminer FR 46-107, probably due to impaired monoterpene biosynthesis of this clone. Gewurztraminer 11-18 Gm and Muscat a petits grains blanc FR 90 skins accumulated significant levels of geraniol, citronellol and nerol derivatives (up to 5.5 mg/kg grape skins) and displayed a heterogenous spectrum of monoterpenes at every stage of ripening. Both Riesling clones produced smaller amounts of the metabolites that were mainly observed at weeks 15-17. In general, the highest concentration of free and bound terpenols was found in the late stages of ripening in all investigated cultivars, whereupon geraniol and its ß-D-glucoside were the predominant terpene metabolites. The ratios of the amount of free to glucosidically bound forms of individual monoterpenes varied considerably at week 15 and 17 post flowering. These values provide a first indication of variable UGT activity in different varieties and/or differential preference of the UGTs for their monoterpene substrates.

Notably, the evolution of monoterpenyl ß-D-glucosides in grape exocarp of the Riesling clones (Table 1) correlated well with the expression pattern of *VvGT14* in the same tissue (Figure 2). While significant transcript levels were only detected at week 11 post flowering remarkable levels of the glucosides were not found until week 13. In contrast, the time course of *VvGT15* mRNA levels in Muscat FR 90 coincided with the terpenyl glucoside concentrations in the same clone as considerable amounts of transcripts and glucosides were found throughout weeks 6 to 17 post flowering. At the very late stages of ripening (weeks 15 to 17) expression of *VvGT16* increased strongly in Gewurztraminer 11-18 Gm, a variety that produced a high concentration of geranyl ß-D-glucosides.

### Example 4

### Heterologous expression of VvGT14, VvGT15 and VvGT16 and enzymatic activity

All methods mentioned in this example were carried out as described in Example 1.

The alleles of *VvGT14a-c, 15a*-*c and 16* were isolated from *V. vinifera* cultivars and cloned in the expression vector pGEX-4T-1. The recombinant proteins were expressed with an N-terminal GST-tag, affinity purified and verified by SDS-PAGE and Western Blot using GST-specific antibody. Enzyme activity studies were performed with UDP-[¹⁴C]glucose and various putative substrates (terpenols, flavonoids and different mono-alcohols) that are known to be glycosidically bound and present in grapes (Gunata et al., 1988).

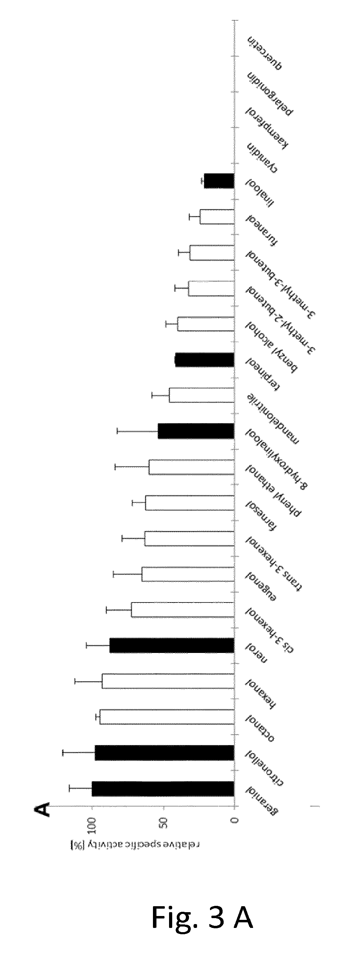

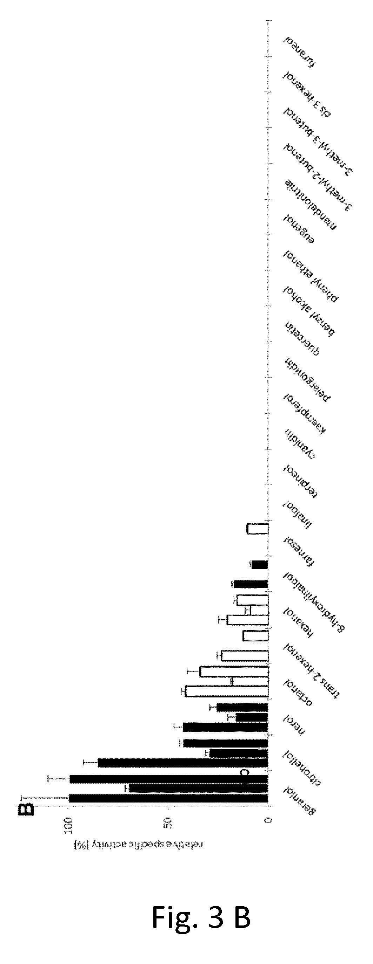

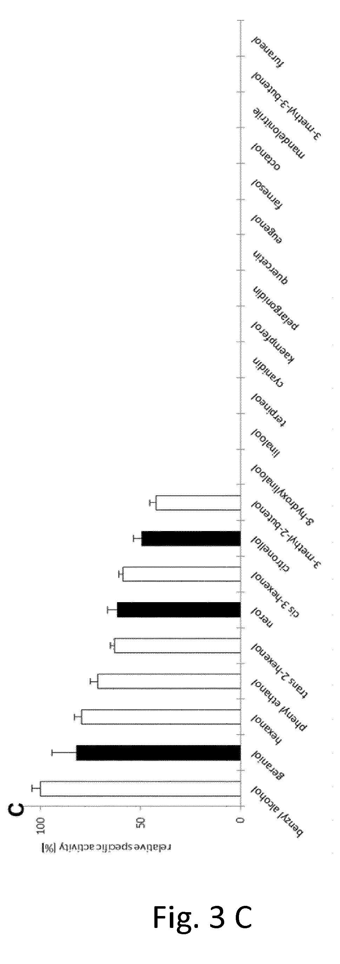

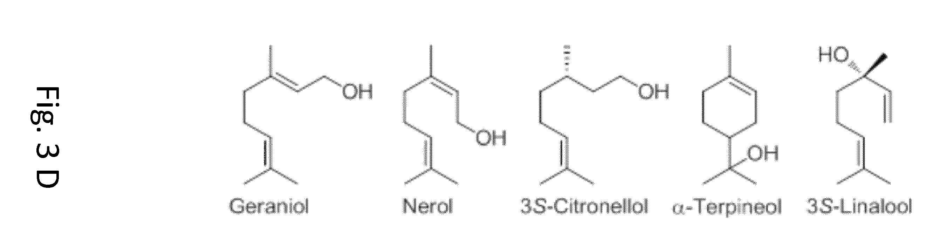

Recombinant VvGT14a, VvGT15a-c and VvGT16 converted several of the tested substrates (Figure 3). VvGT14a preferred geraniol and citronellol but also efficiently (> 80% relative activity) glucosylated nerol, hexanol and octanol. Additionally, VvGT14a showed catalytic activity towards further monoterpenes (terpineol, 8-hydroxylinalool, linalool), short-chain mono-alcohols (3-methyl-2-butenol, 3-methyl-3-butenol, *cis* 3- and *trans* 2-hexenol), benzyl alcohol, phenylethanol, eugenol, farnesol, mandelonitrile, and furaneol. The tested anthocyanidins and flavonoids (cyanidin, pelargonodin, quercetin, and kaempferol,) were not converted at all (< 1%). The three active proteins VvGT15a-c showed a more limited substrate spectrum and glucosylated primarily geraniol, citronellol, nerol, octanol, and hexanol. VvGT15a and c were also able to use 8-hydroxylinalool and *trans* 2-hexenol as acceptor molecule and VvGT15a had low activity for farnesol. Other tested substrates were not converted. VvGT16 showed highest activity towards benzyl alcohol, geraniol and hexanol (> 80% relative activity). Additionally, the protein transformed the terpenoids citronellol and nerol as well as phenylethanol, 3-methyl-2-butenol, *trans* 2-hexenol and *cis* 3-hexenol.

The K_{M} and k_{cat} values of VvGT14a for geraniol (9 µM; 0.02 sec⁻¹), citronellol (9 µM; 0.02 sec⁻¹), nerol (10 µM; 0.02 sec⁻¹) and UDP-glucose (16 µM; 0.03 sec⁻¹) were alike (Table 2) and resembled the kinetic data of VvGT1 (Offen et al., 2006) and CaUGT2 (*Catharanthus roseus*) (Masada et al., 2007) for their natural substrates quercetin (31 µM; 0.075 sec⁻¹) and curcumin (43.9 µM; 0.0165 sec⁻¹), respectively. The data were also similar to those of VvGT5 (5.6 µM; 7.16 sec⁻¹), VvGT6 (9.24 µM; 0.76) and UGT71G1 (57 µM; 0.0175 sec⁻¹) from *Medicago truncatula* (He et al., 2006) for the conversion of quercetin (Ono et al., 2010). Hence, the specificity constants (k_{cat}/K_{M}) identified the monoterpenols as most probable *in vivo* substrates of VvGT14a as the k_{cat}/K_{M} values of geraniol, citronellol and nerol are 4, 4, and 40 fold higher, respectively than the values of the monoterpenols for UGT85B1 from *Sorghum bicolor* (Hansen et al., 2003) (see Table 5). Thus, VvGT14a shows unprecedented glucosyltransferase activity toward geraniol, citronellol and nerol.

The sequence comparison of the active VvGT14a allele with the two inactive alleles showed one point mutation at position 391 (P391L) in VvGT14b and a deletion of 21 aa at position 165 in VvGT14c rendering them inactive (Figure 11). SEQ ID NO: 1 corresponds to the protein sequence of VvGT14a. Proline in position 391 is located in the PSPG motif plant secondary product glycosyltransferase) and is quite conserved among UGTs. The exchange of proline to leucine in VvGT14b leads to an inactive enzyme and has not been reported, yet.

The allelic forms of VvGT15a-c showed a similar substrate spectrum (Figure 3). Remarkably, these alleles had a distinct preference for the monoterpenes geraniol, citronellol and nerol as comparison with the accepted substrates of VvGT14a clearly demonstrated. The kinetics identified geraniol as superior substrate (Table 2). The turnover numbers of VvGT15a-c for the glucosylation activity of geraniol (0.12, 0.1, and 0.17 sec⁻¹, respectively) were 3 to 6-fold higher and the Michaelis constants (63, 81, and 43 µM, respectively) about 2-fold greater than the k_{cat} and K_{M} values for nerol and S-citronellol. This resulted in a highest enzyme specificity constant of VvGT15a-c for geraniol and confirmed the data of the substrate screening (Figure 3) whereas allele c was the most effective (k_{cat}/ K_{M} 3.9 sec⁻¹ mM⁻¹). All three alleles showed a 30 to 40- fold lower turnover number for 8-hydroxylinalool compared to geraniol, although the K_{M} value was similar to the ones obtained for S-citronellol and nerol.

Notably, the glucosylation of monoterpenols by UGT85B1, the cyanohydrins (mandelonitrile) GT from *Sorghum bicolor,* can be seen as promiscuous activity (Hansen et al., 2003). However, the K_{M} data of VvGT14a and VvGT15a-c were 1.6 to 125-fold lower and the k_{cat}/K_{M} values up to 78 times higher than the data obtained for the glucosylation of terpenoids by UGT85B1. Mandelonitrile was only a poor substrate of VvGT14a and was not accepted at all by VvGT15a-c.

VvGT16 glucosylated monoterpenols, some short-chained and aromatic alcohols, albeit with low efficiency (Figure 3, Table 2). Interestingly, benzyl alcohol, an alcohol which has been frequently detected in hydrolysates of glycosides from grape, showed the highest relative activity. However, the low k_{cat}/K_{M} values argue against a role of VvGT16 in the glucosylation of these compounds *in planta.*

The formation of monoterpenyl glucosides was confirmed by LC-MS analysis in comparison with chemically synthesized glucosides (Figure 4). The retention times and fragmentation patterns of the reference material and products formed by VvGT14a, 15a and 16 were identical and in accordance with the proposed fragmentation mechanism (Domon and Costello, 1988; Cole et al., 1989; Salles et al., 1991).

Besides, selected glucosides of the transformed terpenoids were visualized by radio-TLC (Figures 9 and 10). The extracted radioactivity of the enzyme assays consisted exclusively of the terpenyl mono-glucosides, except when 8-hydroxylinalool was used. It seemed that this monoterpene diol is glucosylated at both hydroxyl groups as two spots, presumably the mono-and diglucoside appeared on the radio-TLC plate. The two allelic enzymes VvGT14b and 14c were unable to glycosylate any of the tested substrates. The alignment of the three VvGT14 alleles showed that VvGT14c has an internal deletion of 21 amino acids at position 165 (Figure 11), while VvGT14a and b differed in a single position (P391L).

### Example 5

### Enantioselectivity of VvGT14a, VvGT15a-c and VvGT16

All methods mentioned in this example were carried out as described in Example 1.

Grape berries accumulate free and bound S-citronellol and S-linalool, albeit in lower levels than nerol and geraniol (Table 1). To elucidate the enantiomeric preference of VvGT14a and VvGT15a, racemic citronellol was used as substrate and racemic linalool was transformed by VvGT14a. Chiral phase GC-MS analysis of liberated citronellol after acid hydrolysis of citronellyl ß-D-glucoside demonstrated no enantio-discrimination by VvGT14a and 15a if the reaction mixture is incubated for a prolonged time (24 h; Figure 7). Nevertheless, VvGT15a-c and VvGT14a preferred *S*- over *R*-citronellol (1/0.4 and 1/0.8, respectively) when choosing short incubation times for kinetic assays whereas VvGT16 transformed *R*- and *S*-citronellol with the same efficiency in radiochemical assays (Figure 12). Accordingly, the kinetic data for VvGT15a-c were calculated for S-citronellol (Table 2).

Furthermore, liberated linalool, after enzymatic hydrolysis of linaloyl ß-D-glucoside (formed by VvGT14a), showed a slight enrichment of the *R*-enantiomer (Figure 7). It is important to mention that the hydrolysis of "racemic" (1:1 diasteromeric mixture) linaloyl ß-D-glucoside by AR 2000 revealed no enantio-discrimination by the action of this enzyme, which confirmed the results of previous works in the literature. Thus, VvGT14a and VvGT15a-c show low enantioselectivity towards the *S*-enantiomer of citronellol and VvGT14a towards *R-*linalool during short-term assays. The enantiomers of racemic monoterpenes react with different reaction rates in the enzymatic reactions with a VvGT14a and VvGT15a-c.

While VvGT14a and VvGT15a-c preferentially glucosylated *S*-citronellol in short time assays enantioselectivity of these enzymes for citronellol was not observe in long term studies (Figure 5; Figure 12). This effect is characteristic for studies on the kinetic resolution of racemates. The reaction slows down at 50% conversion, when the fast reacting enantiomer is almost consumed and only the slow reacting counterpart is gradually transformed. Thus, the enantiomeric excess of the product peaks at 50% transformation and then decreases slowly but steadily. In contrast, VvGT14a preferred *R*- over *S*-linalool even in long term assays. The slight preference for *R*-linalool explained the previously observed enrichment of this enantiomer in the glycosidically bound fraction of linalool in Morio Muskat and Muscat Ottonel berries relative to the free fraction. Hence, the moderate enantioselectivities of VvGT14a and 15a-c were in accordance with their proposed biological role as the availability of highly enriched S-citronellol and S-linalool mainly determined the diastereomeric ratio of the glucosidic product.

### Example 6

### Biochemical characterization of VvGT14a, VvGT15a-c and VvGT16

All methods mentioned in this example were carried out as described in Example 1.

The assay conditions were optimized for the conversion of geraniol to determine the kinetic constants of the active enzymes. The highest activity of VvGT14a, 15a-c and 16 was found in Tris-HCl buffer (pH 8.5, 7.5 and 8.5, respectively) at 30 °C. The product formation of VvGT14a (0.2 µg purified enzyme) was linear for at least 90 minutes. K_{M} and k_{cat} values were obtained for geraniol, citronellol, nerol, terpineol, 8-hydroxylinalool, and linalool with a constant UDP-glucose level (108 µM) and for UDP-glucose with a fixed geraniol concentration (100 µM; Table 2). The kinetic parameters were determined from a hyperbolic Michaelis-Menten saturation curves. Due to the low conversion rates of 8-hydroxylinalool, terpineol and linalool, the amount of purified enzyme was increased (2, 2, and 10 µg protein, respectively).

Interestingly, the K_{M} and k_{cat} value of VvGT14a for citronellol. geraniol, nerol and UDP-glucose was quite similar (K_{M} 9-10 µM; k_{cat} 0.02 sec⁻¹; k_{cat}/K_{M} 2.0-2.6 sec⁻¹ mM⁻¹), while the kinetic data for 8-hydroxylinalool, terpineol and linalool explained the significantly lower enzyme activity towards these substrates. The kinetic data of VvGT15a-c (0.5 or 1 µg purified enzyme) were maintained for geraniol, S-citronellol, nerol and 8-hydroxylinalool with a fixed UDP-glucose amount (833 µM) and for UDP-glucose with a constant geraniol concentration (1.25 mM; Table 2). The formation of geranyl, neryl, and citronellyl ß-D-glucoside was linear for at least 10 minutes (VvGT15c) and 20 minutes (VvGT15a and b), but was extended for 8-hydroxylinaloyl ß-D-glucoside up to 30 min (VvGT15c) and 60 min (VvGT15a and b). The kinetic data confirmed the high enzymatic activity of the VvGT15 alleles towards geraniol and UDP-glucose. The K_{M} and k_{cat} value for S-citronellol and nerol was similar, whereas 8-hydroxylinalool was a poor substrate. Furthermore, the data illustrated that VGT15c is superior to VvGT15a and b. Kinetics of VvGT16 were calculated for geraniol, citronellol and nerol (Table 2). Product formation of VvGT16 (5 µg purified protein) was linear for at least 4 hours. K_{M} and k_{cat} values were obtained for the monoterpenes with a constant UDP-glucose level (512.5 µM) and for UDP-glucose with a fixed geraniol concentration (1.25 mM). VvGT16 exhibited the lowest enzymatic activity towards the substrates of the tested UGTs.

### Example 7

### Identification of the natural substrates of VvGT14a, VvGT15a-c and VvGT16

All methods mentioned in this example were carried out as described in Example 1.

To reveal the natural substrates of VvGT14a, VvGT15a-c and VvGT16 (Figure 6), glycosides were isolated by solid phase extraction from grape skins (Gewurztraminer 11-18 Gm) and blooms (Muscat FR90) as they showed highest expression levels of the target genes. An aglycone library of the two tissues was obtained by enzymatic hydrolysis of the glycosides followed by liquid-liquid extraction of the released alcohols and acids. This physiologic library which contained potential natural substrates of UGTs was screened with recombinant VvGTs and either radiochemically labeled or unlabeled UDP-glucose. The formed glycosides were separated by thin layer chromatography (TLC) and visualized by radiodetection whereas identification could be achieved by LC-MS and NMR analysis and, after hydrolysis, by GC-MS and LC-MS (Figure 6).

Initially, the aglycone extracts were incubated with the purified recombinant enzymes (VvGT14a, 15a, and 16) using radiolabeled UDP-[¹⁴C] glucose. Formed products were extracted; radioactivity was quantified by liquid scintillation counting and analyzed by radio-TLC (Figure 7). Screening of the aglycone library obtained from grape skins by VvGT14a and 15a yielded products that showed identical chromatographic properties as geranyl ß-D-glucoside. Enzymatic hydrolysis of the glucosides formed by VvGT14a liberated a substantial amount of geraniol but also remarkable quantities of citronellol, nerol, benzyl alcohol, phenylethanol, and linalool (Figure 7). A similar result was gained with VvGT15a. VvGT16 did not form a visible product neither by using the berry nor from the bloom extracts. The extract from bloom was only tested with VvGT16. Thus, the aglycone library clearly enabled the detection and identification of the natural substrates of VvGT14a and VvGT15a and confirmed the role of these enzymes during grape berry ripening.

VvGT14a and VvGT15a-c readily formed radiolabeled products when incubated with an aglycone library obtained from grape skins of Gewurztraminer 11-18 Gm (Figure 7A). Geranyl and minor amounts of citronellyl and neryl glucoside (as their free alcohols after hydrolysis) were identified as products of VvGT14a and VvGT15a-c (Figure 7B). In contrast, VvGT16 did not form a glucosylated product although expression level of *VvGT16* and amounts of monoterpenyl glucosides were extraordinary high in berry skins in the late stages of ripening and blooms (Gewurztraminer 11-18 Gm). However, additional UDP-sugars, besides UDP-glucose, were not tested as putative donor molecules and the possible formation of di- and triglycosides of one aglycone was not taken into account (Gunata et al., 1988).

The features of the present invention disclosed in the specification, the claims, and/or in the accompanying drawings may, both separately and in any combination thereof, be material for realizing the invention in various forms thereof.

| **Table 1.** Amounts of free Monoterpenes and Monoterpene-ß-D-Glucosides in Grape Skins during Grape Ripening | | | | | | | | |
|---|---|---|---|---|---|---|---|---|
| Weeks post flowering | | | 6 | 9 | 11 | 13 | 15 | 17 |
| White Riesling 239-34 Gm | Linalool | free | n.d. | n.d. | n.d. | n.d. | 0.13 ±0.18 | n.d. |
| | | ß-D-Glucosides | n.d. | n.d. | n.d. | 0.10 ±0.08 | 0.17±0.01 | 1.71 ±0.54 |
| | Nerol | free | n.d. | n.d. | n.d. | n.d. | n.d. | n.d. |
| | | ß-D-Glucosides | n.d. | n.d. | n.d. | n.d. | n.d. | 0.05 ±0.03 |
| | Geraniol | free | n.d. | n.d. | 0.09±0.01 | 0.25 ±0 | 0.26±0.15 | n.d. |
| | | ß-D-Glucosides | n.d. | n.d. | n.d. | n.d. | 0.06 ±0.02 | 0.14 ±0.06 |
| | Citronellol | free | n.d. | n.d. | 0.03 ±0.01 | 0.05 ±0.01 | n.d. | n.d. |
| | | ß-D-Glucosides | n.d. | n.d. | n.d. | 0.03 ±0.02 | n.d. | n.d. |
| White Riesling 24-196 Gm | Linalool | free | n.d. | n.d. | n.d. | n.d. | 0.11 ±0 | n.d. |
| | | ß-D-Glucosides | n.d. | n.d. | n.d. | 0.07 ±0.02 | 0.27 ±0.02 | 0.61 ±0.1 |
| | Nerol | free | n.d. | n.d. | n.d. | n.d. | 0.08 ±0 | n.d. |
| | | ß-D-Glucosides | n.d. | n.d. | n.d. | n.d. | 0.01 ±0.01 | n.d. |
| | Geraniol | free | 0.11 ±0.03 | 0.06 ±0 | 0.17 ±0 | 0.24 ±0.02 | 0.48 ±0.01 | 0.67 ±0.04 |
| | | ß-D-Glucosides | n.d. | n.d. | n.d. | 0.02 ±0 | 0.07 ±0.02 | 0.14 ±0.02 |
| | Citronellol | free | 0.05 ±0 | n.d. | n.d. | 0.06 ±0.02 | 0.09 ±0.01 | n.d. |
| | | ß-D-Glucosides | n.d. | n.d. | n.d. | n.d. | n.d. | n.d. |
| Gewurztraminer FR 46-107 | Linalool | free | - | n.d. | n.d. | n.d. | - | n.d. |
| | | ß-D-Glucosides | - | n.d. | n.d. | n.d. | - | n.d. |
| | Nerol | free | - | n.d. | n.d. | n.d. | - | n.d. |
| | | ß-D-Glucosides | - | n.d. | n.d. | n.d. | - | 0.01 ±0.01 |
| | Geraniol | free | - | n.d. | 0.11 ±0.01 | n.d. | - | 0.20 ±0.01 |
| | | ß-D-Glucosides | - | 0.02 ±0.02 | n.d. | 0.01 ±0.002 | - | 0.03 ±0.04 |
| | Citronellol | free | - | n.d. | n.d. | n.d. | - | n.d. |
| | | ß-D-Glucosides | - | n.d. | n.d. | n.d. | - | n.d. |
| Gewurztraminer 11-18 Gm | Linalool | free | n.d. | n.d. | n.d. | n.d. | n.d. | n.d. |
| | | ß-D-Glucosides | n.d. | 0.1 ±0.04 | n.d. | n.d. | n.d. | n.d. |
| | Nerol | free | n.d. | n.d. | 0.13 ±0.02 | 0.71±0.04 | 1.25 ±0.15 | 1.75 ±0.05 |
| | | ß-D-Glucosides | n.d. | n.d. | 0.06 ± 0.01 | 0.23±0.11 | 0.72 ±0.22 | 0.87 ±0.2 |
| | Geraniol free | free | 0.44 ±0.06 | 0.88 ±0.06 | 0.81 ±0.07 | 2.62 ±0.38 | 3.47 ±0.66 | 5.26 ±0.52 |
| | | ß-D-Glucosides | 0.08 ±0 | 0.04 ±0.01 | 0.18 ±0.01 | 0.55 ±0.16 | 1.72 ±0.18 | 1.53 ±0.4 |
| | Citronellol | free | 0.12 ±0.03 | 0.31 ±0.01 | 0.27±0.02 | 0.49±0.01 | 0.48 ±0.1 | 0.51 ±0.02 |
| | | ß-D-Glucosides | 0.09 ±0.01 | 0.08 ±0.02 | 0.12 ±0.01 | 0.21 ±0.06 | 0.48 ±0.02 | 0.29±0.06 |
| Muscat a petits grains blanc FR 90 | Linalool | free | 0.06 ±0.02 | n.d. | n.d. | 0.13 ±0.02 | 3.03 ±0.01 | 0.62 ±0.12 |
| | | ß-D-Glucosides | 0.11 ±0 | 0.12 ±0.03 | n.d. | 0.05 ±0.01 | 0.27 ±0.10 | 0.37 ±0.16 |
| | Nerol | free | n.d. | n.d. | 0.16 ±0.03 | 0.31 ±0.04 | 0.78 ±0.15 | 0.94 ±0.05 |
| | | ß-D-Glucosides | 0.03 ±0 | 0.04 ±0.02 | n.d. | 0.09 ±0.02 | 0.47 ±0.05 | 0.57 ±0.10 |
| | Geraniol | free | 0.18 ±0.04 | 0.53 ±0.06 | 1.64 ±0.62 | 1.00 ±0.21 | 1.20 ±0.21 | 1.21 ±0.13 |
| | | ß-D-Glucosides | 0.03 ±0 | 0.07 ±0.03 | 0.05 ±0.03 | 0.09 ±0.02 | 0.45 ±0.09 | 0.24 ±0.08 |
| | Citronellol | free | 0.09 ±0.03 | 0.11 ±0.01 | 0.20 ±0.06 | 0.15 ±0.01 | 0.21 ±0 | 0.23 ±0 |
| | | ß-D-Glucosides | n.d. | 0.07 ±0.04 | 0.04 ±0.02 | 0.04 ±0.01 | 0.12 ±0.05 | n.d. |
| Plant material was prepared and analyzed as described in Methods. Grapes were collected during grape ripening at the indicated weeks post flowering. | | | | | | | | |
| n.d.: not detected, -: not determined, Amounts are listed in mg/kg grape skins. n=2 | | | | | | | | |

| **Table 2 .** Kinetics of VvGT14, 15a-c and 16. | | | | |
|---|---|---|---|---|
| enzyme | Substrate | K_{M} [µM] | k_{cat} [sec⁻¹] | k_{cat}/K_{M} [sec⁻¹mM⁻¹] |
| VvGT14 | | | | |
| | citronellol (rac) | 9±0.3 | 0.02 | 2.5 |
| | Geraniol | 9±1.2 | 0.02 | 2.6 |
| | Nerol | 10 ± 0.7 | 0.02 | 2.0 |
| | 8-hydroxylinalool | 48 ± 2.0 | 0.002 | 0.03 |
| | Terpineol | 33 ± 4.4 | 0.003 | 0.1 |
| | linalool (rac) | 47 ± 0.1 | 0.0003 | 0.01 |
| | UDP-glucose | 16 ± 0.03 | 0.03 | 1.6 |
| VvGT15a | | | | |
| | S-citronellol | 29 ± 3.0 | 0.02 | 0.9 |
| | Geraniol | 63 ± 2.4 | 0.12 | 1.9 |
| | Nerol | 48 ± 1.7 | 0.04 | 0.7 |
| | 8-hydroxylinalool | 32 ± 1.4 | 0.003 | 0.1 |
| | UDP-glucose | 49 ± 12.0 | 0.18 | 3.7 |
| VvGT15b | | | | |
| | S-citronellol | 55 ± 1.3 | 0.03 | 0.6 |
| | Geraniol | 81 ± 1.0 | 0.10 | 1.2 |
| | Nerol | 40 ± 3.7 | 0.03 | 0.8 |
| | 8-hydroxylinalool | 33 ± 1.8 | 0.003 | 0.1 |
| | UDP-glucose | 43 ± 1.0 | 0.17 | 4.1 |
| VvGT15c | | | | |
| | S-citronellol | 20 ± 1.6 | 0.03 | 1.8 |
| | Geraniol | 43 ± 0.7 | 0.17 | 3.9 |
| | Nerol | 28 ± 0.9 | 0.06 | 2.2 |
| | 8-hydroxylinalool | 17 ± 0.2 | 0.004 | 0.3 |
| | UDP-glucose | 51 ± 1.6 | 0.26 | 5.0 |
| VvGT16 | | | | |
| | citronellol (rac) | 108 ± 2.5 | 0.008 | 0.07 |
| | Geraniol | 355 ± 14 | 0.015 | 0.04 |
| | Nerol | 118 ± 4.7 | 0.009 | 0.08 |
| | UDP-glucose | 149 ± 10 | 0.013 | 0.09 |

**Table 3. Gene specific primers used for GeXP. All primers are chimeric containing a universal tag sequence at their 5'-end (uncapitalized). The final concentration of each reverse primer in the GeXP reverse transcription reaction is given under conc. The CRIBI-Genomics accession numbers (http://genomes.cribi.unipd.it) are given under locus ID.**

| **gene** | **locus ID** | **primer** | **Sequence** | **conc.** | **product** | **SEQ ID NO** |
|---|---|---|---|---|---|---|
| VvActin | VIT_04s0044g00580 | for | aggtgacactatagaataCTTGCATCCCTCAGCACCTT | | | SEQ ID NO: 3 |
| VvActin | VIT_04s0044g00580 | rev | gtacgactcactatagggaTCCTGTGGACAATGGATGGA | 0.1 nM | 119 bp | SEQ ID NO: 4 |
| VvAP47 | VIT_02s0012g00910 | for | aggtgacactatagaataTGTTGTTGAGGCTGTTGCGCTGT | | | SEQ ID NO: 5 |
| VvAP47 | VIT_02s0012g00910 | rev | gtacgactcactatagggaAGGCCCTCTCTCCTCCAACCAA | 1 nM | 304 bp | SEQ ID NO: 6 |
| VvPP2A | VIT_01s0011g03280 | for | aggtgacactatagaataAGGGTTGTGCCACACTGGGC | | | SEQ ID NO: 7 |
| VvPP2A | VIT_01s0011g03280 | rev | gtacgactcactatagggaGCAACCATGTAGCGAACACGCC | 2 nM | 152 bp | SEQ ID NO: 8 |
| VvSAND | VIT_06s0004g02820 | for | aggtgacactatagaataACCCCTTTGCTCGGAGGAACAGA | | | SEQ ID NO: 9 |
| VvSAND | VIT_06s0004g02820 | rev | gtacgactcactatagggaACCTGAAGCTTGCCTTGTCGC | 2 nM | 166 bp | SEQ ID NO: 10 |
| VvTIP41 | VIT_03s0091g00270 | for | aggtgacactatagaataGCTACCGGAAACCAGCGGGC | | | SEQ ID NO: 11 |
| VvTIP41 | VIT_03s0091g00270 | rev | gtacgactcactatagggaGCAATCCATGCCGTCCATCCGT | 1 nM | 144 bp | SEQ ID NO: 12 |
| VvGT14 | VIT_18s0001g06060 | for | aggtgacactatagaataTGTGGCTTCATGGCCTATGTGCA | | | SEQ ID NO: 13 |
| VvGT 14 | VIT_18s0001g06060 | rev | gtacgactcactatagggaCCGGAAGTAGCTGAAGAGGACCA | 12.5 nM | 374 bp | SEQ ID NO: 14 |
| VvGT15 | VIT_06s0004g05780 | for | aggtgacactatagaataTCCGGACGGCTTATCTGATGGCC | | | SEQ ID NO: 15 |
| VvGT15 | VIT_06s0004g05780 | rev | gtacgactcactatagggaTGAGGGGATGAAGCTCAGGCACTG | 50 nM | 363 bp | SEQ ID NO: 16 |
| VvGT16 | VIT_03s0017g01130 | for | aggtgacactatagaataGCAGTTTCGCCCTCTTATTGATGGA | | | SEQ ID NO: 17 |
| VvGT16 | VIT_03s0017g01130 | rev | gtacgactcactatagggaAGCTTTGGAAGCACTGTCCGTT | 50 nM | 248 bp | SEQ ID NO: 18 |

**Table 4. Primers used in PCR for subsequent cloning and sequencing. The CRIBI-Genomics accession numbers (http://genomes.cribi.unipd.it) are given under locus ID.**

| **gene** | **locus ID** | **primer** | **sequence** | **SEQ ID NO** |
|---|---|---|---|---|
| VvGT14 | VIT_18s0001g06060 | for | AATGGGTTCCATGGAGAAGCC | SEQ ID NO: 19 |
| VvGT14 | VIT_18s0001g06060 | rev | GGAAAGTTGAGATCTAGAGAAGC | SEQ ID NO: 20 |
| VvGT15 | VIT_06s0004g05780 | for | TCACCTCTCAACTCTTCCACG | SEQ ID NO: 21 |
| VvGT15 | VIT_06s0004g05780 | rev | GAGTTTGGTTGTACACAACTCTG | SEQ ID NO: 22 |
| VvGT16 | VIT_03s0017g01130 | for | TCCTCCCCAGAGTGCAAG | SEQ ID NO: 23 |
| VvGT16 | VIT_03s0017g01130 | rev | TGACCAACTCCTATAACAG | SEQ ID NO: 24 |

**Table 5. Comparison of different plant glycosyl transferases with respect to their glucosyl transferase activity (k_{cat} /K_{M} [s⁻¹M⁻¹]) for nerol, geraniol and citronellol.**

| **Glycosyl transferase** | **Organism** | **Substrate** | **k_{cat}/K_{M}[s⁻¹M⁻¹]** |
|---|---|---|---|
| VvGT7 | *V*. *vinifera* | Nerol | 7.0 |
| | | Geraniol | 1.0 |
| | | Citronellol | 1.0 |
| VvGT14 | *V*. *vinifera* | Nerol | 2000 |
| | | Geraniol | 2600 |
| | | Citronellol | 2500 |
| VvGT15 | *V*. *vinifera* | Nerol | 2200 |
| | | Geraniol | 3900 |
| | | Citronellol | 1800 |
| VvGT16 | *V*. *vinifera* | Nerol | 0.08 |
| | | Geraniol | 0.04 |
| | | Citronellol | 0.07 |
| UGT85B1 | *Sorghum bicolor* (millet) | Nerol | 50 |
| | | Geraniol | 690 |
| | | Citronellol | 690 |

| | | | |
|---|---|---|---|
| (Recombinant VvGT7 glycosyl transferase protein (VIT_16s0050g01580) was prepared and analyzed by the same methods as described above for VvGT14-16.) | | | |

### References

Bowles, D., Lim, E.K., Poppenberger, B., Vaistij, F.E. (2006) Glycosyltransferases of lipophilic small molecules. Annu. Rev. Plant Biol. 57: 567-597.
Cole, R.B., Tabet, J.C., Salles, C., and Crouzet, J. (1989). Structural "memory effects" influencing decompositions of glucose alkoxide anions produced from monoterpene glycoside isomers in tandem mass spectrometry. Rapid Commun. Mass Spectrom. 3: 59-61.
Domon, B. and Costello, C.E. (1988). A systematic nomenclature for carbohydrate fragmentations in FAB-MS/MS spectra of glycoconjugates. Glycoconjugate 5: 397-409.
Gunata, Z., Bitteur, S.M., Brillouet, J.-M., Bayonove, C.L., and Cordonnier, R. (1988). Sequential enzymic hydrolysis of potenially aromatic glycosides from grape. Carbohydr. Res. 184: 139-149.
Hansen, K.S., Kristensen, C., Tattersall, D.B., Jones, P.R., Olsen, C.E., Bak, S., and Moller, B.L. (2003). The in vitro substrate regiospecificity of recombinant UGT85B1, the cyanohydrin glucosyltransferase from Sorghum bicolor. Phytochemistry 64: 143-151.
He, X.Z., Wang, X., and Dixon, R.A. (2006). Mutational analysis of the Medicago glycosyltransferase UGT71G1 reveals residues that control regioselectivity for (iso)flavonoid glycosylation. J. Biol. Chem. 281: 34441-34447.
Jesus Ibarz, M., Ferreira, V., Hernández-Orte, P., Loscos, N., and Cacho, J. (2006). Optimization and evaluation of a procedure for the gas chromatographic-mass spectrometric analysis of the aromas generated by fast acid hydrolysis of flavor precursors extracted from grapes. J. Chromatogr. A 1116: 217-229.
Masada, S., Terasaka, K., and Mizukami, H. (2007). A single amino acid in the PSPG-box plays an important role in the catalytic function of CaUGT2 (Curcumin glucosyltransferase), a Group D Family 1 glucosyltransferase from Catharanthus roseus. FEBS Lett. 581: 2605-2610.
Mateo, J., and Jiménez, M. (2000). Monoterpenes in grape juice and wines. J. Chromatogr. A 881: 557-567.
Offen, W., Martinez-Fleites, C., Yang, M., Kiat-Lim, E., Davis, B.G., Tarling, C.A., Ford, C.M., Bowles, D.J., and Davies, G.J. (2006). Structure of a flavonoid glucosyltransferase reveals the basis for plant natural product modification. EMBO J. 25: 1396-1405.
Ono, E., Homma, Y., Horikawa, M., Kunikane-Doi, S., Imai, H., Takahashi, S., Kawai, Y., Ishiguro, M., Fukui, Y., and Nakayama, T. (2010). Functional differentiation of the glycosyltransferases that contribute to the chemical diversity of bioactive flavonol glycosides in grapevines (Vitis vinifera). Plant Cell 22: 2856-2871.
Piñeiro, Z., Palma, M., and Barroso, C.G. (2004). Determination of terpenoids in wines by solid phase extraction and gas chromatography. Anal. Chim. Acta 513: 209-214.
Reid, K.E., Olsson, N., Schlosser, J., Peng, F., and Lund, S.T. (2006). An optimized grapevine RNA isolation procedure and statistical determination of reference genes for real-time RT-PCR during berry development. BMC Plant Biol. 6: 27.
Salles, C., Jallageas, J.-C., Beziat, Y., and Cristau, H.-J. (1991). Synthesis of 4- and 10-deuterated neryl and geranyl-β-D-glucosides and their use in corroboration of a mechanism proposed for the fragmentation of heterosides in tandem mass spectrometry. J. Label. Compd. Radiopharm. 31: 11-22.
Skouroumounis, G.K. and Sefton, M.A. (2000). Acid-catalyzed hydrolysis of alcohols and their β-D-glucopyranosides. J. Agric. Food Chem. 48: 2033-2039.
Williams, P.J., Strauss, C.R., Wilson, B., and Massy-Westropp, R.A. (1982). Studies on the hydrolysis of Vitis vinifera monoterpene precursor compounds and model monoterpene ß-D-glucosides rationalizing the monoterpene composition of grapes. J. Agric. Food Chem. 30: 1219-1223.
Wilson, B, Strauss, C.R. and Williams, P.J. (1986). The distribution of free and glycosidically bound monoterpenes among skin, juice, and pulp fractions of some white grape varieties. Am. J. Enol. Vitic. 37: 107-111.
Zeng, Y., and Yang, T. (2002). RNA isolation from highly viscous samples rich in polyphenols and polysaccharides. Plant Mol. Biol. Rep. 20: 417.

## Claims

1. Glycosyl transferase having an amino acid sequence that
a) comprises the sequence of SEQ ID NO: 1; or
b) comprises a sequence that is at least 90%, preferably at least 95%, more preferably at least 98%, identical to SEQ ID NO: 1; or
c) comprises a part of the sequence of SEQ ID NO: 1, wherein said part of the sequence of SEQ ID NO: 1 is at least 50, preferably at least 80, more preferably at least 100, more preferably at least 200, amino acids in length; or
d) comprises a sequence that is at least 90%, preferably at least 95%, more preferably at least 98%, identical to a part of the sequence of SEQ ID NO: 1, wherein said part of the sequence of SEQ ID NO: 1 is at least 50, preferably at least 80, more preferably at least 100, more preferably at least 200, amino acids in length;
e) comprises the sequence of SEQ ID NO: 2; or
f) comprises a sequence that is at least 90%, preferably at least 95%, more preferably at least 98%, identical to SEQ ID NO: 2; or
g) comprises a part of the sequence of SEQ ID NO: 2, wherein said part of the sequence of SEQ ID NO: 2 is at least 50, preferably at least 80, more preferably at least 100, more preferably at least 200, amino acids in length; or
h) comprises a sequence that is at least 90%, preferably at least 95%, more preferably at least 98%, identical to a part of the sequence of SEQ ID NO: 2, wherein said part of the sequence of SEQ ID NO: 2 is at least 50, preferably at least 80, more preferably at least 100, more preferably at least 200, amino acids in length.

2. The glycosyl transferase according to claim 1, wherein the glycosyl transferase is a terpene glycosyl transferase, preferably a monoterpene glycosyl transferase.

3. The glycosyl transferase according to any of the preceding claims, wherein the glycosyl transferase is capable of catalyzing formation of a glycoside in which a sugar is linked to a hydroxy-containing terpene through a β-D-glycosyl linkage and/or formation of a glycose ester in which a sugar is linked to a carboxy-containing terpene through a P-D-glycose ester linkage.

4. The glycosyl transferase according to any of the preceding claims, wherein said glycosyl transferase is capable of using UDP-glucose as sugar donor.

5. The glycosyl transferase according to any of the preceding claims, wherein the glycosyl transferase is capable of catalyzing glycosylation, preferably glucosylation, of geraniol, linalool, citronellol, nerol, 8-hydroxylinalool, farnesol, furaneol, hexanol and/or octanol, preferably geraniol and/or citronellol and/or furaneol, more preferably geraniol.

6. A nucleic acid molecule encoding a glycosyl transferase as defined in any of claims 1-5, wherein, preferably, said nucleic acid molecule is a DNA molecule.

7. A vector comprising a DNA sequence encoding a glycosyl transferase as defined in any of claims 1-5.

8. A host cell containing or transfected with the nucleic acid molecule according to claim 6 or the vector according to claim 7.

9. The host cell according to claim 8, wherein said host cell produces a glycosyl transferase as defined in any of claims 1-5.

10. A transgenic plant comprising a nucleic acid molecule as defined in claim 6 or a vector as defined in claim 7, wherein, preferably, said plant is not a *Vitis vinifera* plant, more preferably not a grape vine.

11. The transgenic plant according to claim 10, wherein said transgenic plant produces a glycosyl transferase as defined in any of claims 1-5.

12. Use of a glycosyl transferase as defined in any of claims 1-5 or a nucleic acid molecule as defined in claim 6 or a vector as defined in claim 7 or a host cell as defined in any of claims 8-9 or a transgenic plant as defined in any of claims 10-11 for producing a terpene glycoside, terpene glycose ester, octanyl glycoside, furaneyl glycoside or hexanyl glycoside.

13. A method of forming
a) a terpene glycoside in which a hydroxy-containing terpene is covalently linked to a sugar group through a glycosidic bond or
b) a terpene glycose ester in which a carboxy-containing terpene is covalently linked to a sugar group through a glycose ester bond or
c) an octanyl glycoside in which octanol is covalently linked to a sugar group through a glycosidic bond or
d) a furaneyl glycoside in which furaneol is covalently linked to a sugar group through a glycosidic bond or
e) a hexanyl glycoside in which hexanol is covalently linked to a sugar group through a glycosidic bond,
said method comprising contacting
a) a hydroxy-containing terpene or
b) a carboxy-containing terpene or
c) octanol or
d) furaneol or
e) hexanol
with a sugar donor and a glycosyl transferase as defined in any of the embodiments above under conditions appropriate for the transfer of the sugar group of said sugar donor to
a) a hydroxyl group of said hydroxy-containing terpene or
b) a carboxyl group of said carboxy-containing terpene or
c) the hydroxyl group of octanol or
d) the hydroxyl group of furaneol or
e) the hydroxyl group of hexanol
under formation of
a) a glycosidic bond between said terpene and said sugar group or
b) an ester bond between said terpene and said sugar group or
c) a glycosidic bond between octanol and said sugar group or
d) a glycosidic bond between furaneol and said sugar group or
e) a glycosidic bond between hexanol and said sugar group,
thereby forming
a) a terpene glycoside or
b) a terpene glycose ester or
c) an octanyl glycoside or
d) a furaneyl glycoside or
e) a hexanyl glycoside.

14. A method of producing a terpene glycoside, terpene glycose ester, octanyl glycoside, furaneyl glycoside or hexanyl glycoside, said method comprising the steps of:
- culturing or growing a host cell as defined in any of claims 8-9 or a transgenic plant as defined in any of claims 10-11; and
- collecting from said host cell or transgenic plant said terpene glycoside, terpene glycose ester, octanyl glycoside, furaneyl glycoside or hexanyl glycoside.

15. A method of producing a protein having glycosyl transferase activity, said method comprising the steps of:
- culturing or growing a host cell as defined in any of claims 8-9 or a transgenic plant as defined in any of claims 10-11; and
- collecting from the host cell or transgenic plant a protein having glycosyl transferase activity.


SEQUENCE LISTING

<110>  Technische Universität München

<120>  Glycosyl Transferases and their Uses

<130>  U30571EP

<160>  26

<170>  PatentIn version 3.5

<210>  1
<211>  473
<212>  PRT
<213>  Vitis vinifera

<400>  1

Met Gly Ser Met Glu Lys Pro His Ala Val Cys Ile Pro Tyr Pro Ala
1               5                   10                  15

Gln Gly His Ile Asn Pro Met Leu Lys Val Ala Lys Leu Leu His Phe
            20                  25                  30

Arg Gly Phe Arg Ile Thr Phe Val Asn Thr Glu Phe Asn His Thr Arg
        35                  40                  45

Leu Leu Lys Ala Gln Gly Pro Asn Ser Leu Asn Gly Leu Pro Thr Phe
    50                  55                  60

Gln Phe Glu Thr Ile Pro Asp Gly Leu Pro Pro Ser Asn Val Asp Ala
65                  70                  75                  80

Thr Gln Asp Ile Pro Ser Leu Cys Ala Ser Thr Lys Lys Asn Cys Leu
                85                  90                  95

Ala Pro Phe Arg Arg Leu Leu Ala Lys Leu Asn Asp Arg Gly Pro Pro
            100                 105                 110

Val Thr Cys Ile Phe Ser Asp Ala Val Met Ser Phe Thr Leu Asp Ala
        115                 120                 125

Ala Gln Glu Leu Gly Ile Pro Asp Leu Leu Leu Trp Thr Ala Ser Ala
    130                 135                 140

Cys Gly Phe Met Ala Tyr Val Gln Tyr Arg Ser Leu Ile Asp Lys Gly
145                 150                 155                 160

Phe Thr Pro Leu Lys Asp Glu Ser Tyr Leu Thr Asn Gly Tyr Leu Asp
                165                 170                 175

Thr Val Val Asp Trp Ile Pro Gly Met Lys Gly Ile Arg Leu Lys Asp
            180                 185                 190

Leu Pro Ser Phe Ile Arg Thr Thr Asp Pro Asp Asp Ile Met Leu Asp
        195                 200                 205

Phe Ala Met Gly Glu Leu Glu Arg Ala Arg Lys Ala Ser Ala Ile Ile
    210                 215                 220

Phe Asn Thr Phe Asp Ala Leu Glu Gln Glu Val Leu Asp Ala Ile Ala
225                 230                 235                 240

Pro Met Tyr Pro Pro Ile Tyr Thr Ile Gly Pro Leu Gln Leu Leu Pro
                245                 250                 255

Asp Gln Ile His Asp Ser Glu Leu Lys Leu Ile Gly Ser Asn Leu Trp
            260                 265                 270

Lys Glu Glu Pro Glu Cys Leu Lys Trp Leu Asp Ser Lys Glu Pro Asn
        275                 280                 285

Ser Val Val Tyr Val Asn Tyr Gly Ser Ile Thr Val Met Thr Pro Gln
    290                 295                 300

Gln Leu Ile Glu Phe Ala Trp Gly Leu Ala Asn Ser Asn Gln Ser Phe
305                 310                 315                 320

Leu Trp Ile Leu Arg Pro Asp Leu Val Ser Gly Glu Ser Ala Ile Leu
                325                 330                 335

Pro Pro Glu Phe Val Ala Glu Thr Glu Asp Arg Gly Leu Leu Ala Gly
            340                 345                 350

Trp Cys Pro Gln Glu Gln Val Leu Thr His Gln Ala Ile Gly Gly Phe
        355                 360                 365

Leu Thr His Asn Gly Trp Asn Ser Thr Ile Glu Gly Leu Cys Ala Gly
    370                 375                 380

Val Pro Met Ile Cys Trp Pro Phe Phe Ala Glu Gln Gln Thr Asn Cys
385                 390                 395                 400

Arg Tyr Cys Cys Thr Glu Trp Gly Val Gly Met Glu Ile Asp Ser Asp
                405                 410                 415

Val Lys Arg Asp Glu Val Ala Lys Leu Val Arg Glu Leu Met Val Gly
        420                 425                 430

Glu Lys Gly Lys Val Met Lys Lys Lys Thr Met Glu Trp Lys His Arg
            435                 440                 445

Ala Glu Val Ala Thr Thr Gly Pro Asp Gly Ser Ser Tyr Leu Asn Leu
    450                 455                 460

Glu Lys Ile Phe Glu Gln Val Leu Leu
465                 470

<210>  2
<211>  442
<212>  PRT
<213>  Vitis vinifera

<400>  2

Met Pro Arg Arg Val Val Leu Val Pro Cys Pro Phe Gln Gly His Ile
1               5                   10                  15

Asn Pro Met Leu Gln Leu Gly Ala Ile Leu His Ser Arg Gly Phe Ser
            20                  25                  30

Ile Thr Val Ala His Thr Gln Tyr Asn Ser Pro Asp Pro Ser Asn His
        35                  40                  45

Pro Asp Phe Ser Phe Leu Pro Ile Pro Asp Gly Leu Ser Asp Gly Gln
    50                  55                  60

Asn Phe Ala Ser Leu Leu Asn Leu Val Leu Ala Ala Asn Val Asn Cys
65                  70                  75                  80

Glu Ser Pro Leu Arg Glu Tyr Leu Ala Glu Lys Gln Glu Gln His Gly
                85                  90                  95

Asp Ile Ala Cys Ile Ile His Asp Ile Thr Met Tyr Phe Ala Glu Ala
            100                 105                 110

Val Ala Asn His Leu Lys Val Pro Ser Ile Asn Leu Val Thr Ser Asn
        115                 120                 125

Val Ser Thr Thr Ile Ala His Asn Ala Phe Pro Ser Leu Leu Glu Lys
    130                 135                 140

Gly His Ile Pro Leu Gln Gly Ser Thr Leu His Asp Pro Val Pro Glu
145                 150                 155                 160

Leu His Pro Leu Arg Phe Lys Asp Leu Pro Ile Ser Arg Leu Gly Asp
                165                 170                 175

Leu Glu Val Phe Phe Gln Ile Leu Val Asn Met Tyr Lys Lys Lys Phe
            180                 185                 190

Ser Ser Pro Ile Ile Trp Asn Thr Met Asp Cys Leu Glu Gln Ser Ser
        195                 200                 205

Leu Thr Gln Arg Gln Gln Gln Leu Gln Val Pro Phe Phe Pro Ile Gly
    210                 215                 220

Pro Leu His Lys Leu Ala Pro Pro Ser Ser Ser Ser Leu Leu Glu Glu
225                 230                 235                 240

Asp Ser Ser Cys Ile Thr Trp Leu Asp Lys Gln Ser Pro Lys Ser Val
                245                 250                 255

Ile Tyr Val Ser Trp Gly Ser Leu Ala Cys Met Asp Ala Lys Asp Leu
            260                 265                 270

Ala Glu Val Ala Trp Gly Leu Ala Asn Ser Asn Gln Pro Phe Leu Trp
        275                 280                 285

Val Val Arg Pro Gly Ser Val Arg Gly Ser Gln Trp Ile Glu Gln Leu
    290                 295                 300

Pro Glu Thr Phe Met Asp Thr Val Gly Glu Arg Cys His Ile Val Lys
305                 310                 315                 320

Trp Ala Pro Gln Lys Glu Val Leu Gly His Arg Ala Val Gly Gly Phe
                325                 330                 335

Trp Ser His Cys Gly Trp Asn Ser Thr Leu Glu Ser Ile Ser Glu Gly
            340                 345                 350

Val Pro Met Ile Cys Arg Pro Tyr Ser Gly Asp Gln Arg Val Asn Thr
        355                 360                 365

Arg Tyr Ile Ser His Val Trp Lys Val Gly Leu Glu Leu Glu Ser Asp
    370                 375                 380

Glu Leu Glu Arg Val Glu Ile Glu Arg Ala Val Arg Arg Leu Met Val
385                 390                 395                 400

Asp Gly Glu Gly Glu Glu Met Arg Gln Arg Ala Met Glu Leu Lys Glu
                405                 410                 415

Lys Val Glu Ile Cys Thr Ser Glu Gly Gly Ser Ser Asn Arg Ala Leu
            420                 425                 430

Lys Glu Leu Val Glu Tyr Ile Ser Ser Phe
        435                 440

<210>  3
<211>  38
<212>  DNA
<213>  Artificial Sequence

<220>
<223>  VvActin forward primer

<400>  3
aggtgacact atagaatact tgcatccctc agcacctt                              38

<210>  4
<211>  39
<212>  DNA
<213>  Artificial Sequence

<220>
<223>  VvActin reverse primer

<400>  4
gtacgactca ctatagggat cctgtggaca atggatgga                             39

<210>  5
<211>  41
<212>  DNA
<213>  Artificial Sequence

<220>
<223>  VvAP47 forward primer

<400>  5
aggtgacact atagaatatg ttgttgaggc tgttgcgctg t                          41

<210>  6
<211>  41
<212>  DNA
<213>  Artificial Sequence

<220>
<223>  VvAP47 reverse primer

<400>  6
gtacgactca ctatagggaa ggccctctct cctccaacca a                          41

<210>  7
<211>  38
<212>  DNA
<213>  Artificial Sequence

<220>

<223>  VvPP2A forward primer

<400>  7
aggtgacact atagaataag ggttgtgcca cactgggc                              38


<210>  8
<211>  41
<212>  DNA
<213>  Artificial Sequence

<220>
<223>  VvPP2A reverse primer

<400>  8
gtacgactca ctatagggag caaccatgta gcgaacacgc c                          41


<210>  9
<211>  41
<212>  DNA
<213>  Artificial Sequence

<220>
<223>  VvSAND forward primer

<400>  9
aggtgacact atagaataac ccctttgctc ggaggaacag a                          41


<210>  10
<211>  40
<212>  DNA
<213>  Artificial Sequence

<220>
<223>  VvSAND reverse primer

<400>  10
gtacgactca ctatagggaa cctgaagctt gccttgtcgc                            40


<210>  11
<211>  38
<212>  DNA
<213>  Artificial Sequence

<220>
<223>  VvTIP41 forward primer

<400>  11
aggtgacact atagaatagc taccggaaac cagcgggc                              38


<210>  12
<211>  41
<212>  DNA
<213>  Artificial Sequence

<220>
<223>  VvTIP41 reverse primer

<400>  12

gtacgactca ctatagggag caatccatgc cgtccatccg t 41

<210> 13
<211> 41
<212> DNA
<213> Artificial Sequence

<220>
<223> VvGT14 forward primer

<400> 13
aggtgacact atagaatatg tggcttcatg gcctatgtgc a 41

<210> 14
<211> 42
<212> DNA
<213> Artificial Sequence

<220>
<223> VvGT14 reverse primer

<400> 14
gtacgactca ctatagggac cggaagtagc tgaagaggac ca 42

<210> 15
<211> 41
<212> DNA
<213> Artificial Sequence

<220>
<223> VvGT15 forward primer

<400> 15
aggtgacact atagaatatc cggacggctt atctgatggc c 41

<210> 16
<211> 43
<212> DNA
<213> Artificial Sequence

<220>
<223> VvGT15 reverse primer

<400> 16
gtacgactca ctatagggat gaggggatga agctcaggca ctg 43

<210> 17
<211> 43
<212> DNA
<213> Artificial Sequence

<220>
<223> VvGT16 forward primer

<400> 17
aggtgacact atagaatagc agtttcgccc tcttattgat gga 43

<210>  18
<211>  41
<212>  DNA
<213>  Artificial Sequence

<220>
<223>  VvGT16 reverse primer

<400>  18
gtacgactca ctatagggaa gctttggaag cactgtccgt t                          41


<210>  19
<211>  21
<212>  DNA
<213>  Artificial Sequence

<220>
<223>  VvGT14 forward primer

<400>  19
aatgggttcc atggagaagc c                                                21


<210>  20
<211>  23
<212>  DNA
<213>  Artificial Sequence

<220>
<223>  VvGT14 reverse primer

<400>  20
ggaaagttga gatctagaga agc                                              23


<210>  21
<211>  21
<212>  DNA
<213>  Artificial Sequence

<220>
<223>  VvGT15 forward primer

<400>  21
tcacctctca actcttccac g                                                21


<210>  22
<211>  23
<212>  DNA
<213>  Artificial Sequence

<220>
<223>  VvGT15 reverse primer

<400>  22
gagtttggtt gtacacaact ctg                                              23


<210>  23
<211>  18
<212>  DNA

<213>  Artificial Sequence

<220>
<223>  VvGT16 forward primer

<400>  23
tcctccccag agtgcaag                                                    18


<210>  24
<211>  19
<212>  DNA
<213>  Artificial Sequence

<220>
<223>  VvGT16 reverse primer

<400>  24
tgaccaactc ctataacag                                                   19


<210>  25
<211>  1422
<212>  DNA
<213>  Artificial Sequence

<220>
<223>  DNA sequence coding for the amino acid sequence of Terpene
       Glycosyl Transferase VvGT14

<400>  25
atgggttcca tggagaagcc tcatgcagtt tgcattccat accctgctca ggggcacatc      60

aatcctatgc taaaagtagc aaagctcctc catttcagag gctttcgtat cacctttgtc     120

aacacagagt tcaaccacac acgcttactc aaggcccagg gccccaactc cctcaatggt     180

ctccccacct ttcagttcga aaccattccc gatggtctcc caccgtccaa tgtcgacgca     240

acccaagaca tcccttccct ctgtgcatcc accaaaaaaa actgtttggc tccctttaga     300

cgccttctcg ccaaactcaa cgacagaggt cctcccgtca cttgcatatt ttctgacgct     360

gtcatgagct tcactttaga cgcggctcaa gaactcggca ttcccgacct tcttttatgg     420

acagctagtg cttgtggctt catggcctat gtgcagtatc gcagtctcat cgacaagggt     480

tttacaccac tcaaagatga gagttatcta acaaatgggt atttggatac tgttgttgat     540

tggataccgg gcatgaaagg tatccgtttg aaggatctcc cgagcttcat tcggactacc     600

gatccagatg atatcatgct ggactttgcc atgggtgagc ttgaaagggc tcgcaaggct     660

tctgccatca ttttcaatac gtttgacgct ttagagcagg aggttttgga tgcaattgct     720

cccatgtatc ccccaatcta caccattggt cctcttcagc tacttccgga tcagattcac     780

gatagcgaac taaagttgat tggatcaaat ctgtggaagg aagagcctga gtgtctcaaa     840

tggctggatt ccaaagaacc caactctgtt gtttacgtta attatggaag catcacagtc     900

atgactcccc agcaactgat tgagtttgct tggggacttg caaatagcaa tcaaagcttc     960

ttgtggattc ttaggccaga tctcgtgtct ggtgagtcgg ccattctgcc accagaattc 1020

gtggcagaaa cagaagacag gggtttacta gcaggttggt gtcctcaaga gcaagttctc 1080

acgcaccagg ccattggcgg cttcttaact cataacggct ggaattccac aattgagggt 1140

ttatgcgctg gagtgcctat gatttgttgg ccattttttg cagagcagca aaccaactgt 1200

cgttactgct gcactgagtg gggcgtagga atggagatag acagtgatgt taagagggat 1260

gaagttgcaa agcttgtgag agagttgatg gtgggagaga aaggcaaagt aatgaaaaag 1320

aaaaccatgg agtggaagca tagggcagaa gtggctacca ctggtccaga tggatcatct 1380

tacttgaatt tggagaaaat atttgaacaa gtgcttctct ag 1422

<210> 26
<211> 1329
<212> DNA
<213> Artificial Sequence

<220>
<223> DNA sequence coding for the amino acid sequence of Terpene
      Glycosyl Transferase VvGT15

<400> 26
atgccgagga gagtggtact tgttccatgt ccattccaag gccacataaa tcccatgctt 60

cagctgggag caatactcca ttcaaggggc ttttccatca cagttgctca cacccaatac 120

aactctcctg atccctccaa ccaccctgat ttcagcttcc tccccattcc ggacggctta 180

tctgatggcc aaaactttgc cagtctatta aatcttgtac tggctgctaa tgtcaactgt 240

gaatctcctc tccgggaata cctggcagaa aaacaagaac agcatggcga catcgcttgt 300

ataatccatg atatcaccat gtacttcgct gaggctgtgg ctaaccattt gaaagttcca 360

agyatcaacc tcgtcactag caatgtttcc actaccatag ctcacaatgc ctttcctagc 420

ctcctggaaa aaggtcacat tcccctgcaa ggctctaccc tacatgatcc agtgcctgag 480

cttcatcccc tcagattcaa ggatcttccc atctcccgtt tgggagactt ggaagctttc 540

ttccagatac tagttaatat gtacaaaaag aaattttctt cacccatcat ttggaacacc 600

atggactgcc ttgaacaatc ctcattaaca cagcgacaac aacagctcca ggtcccattc 660

ttcccaatag gccctctcca taaacttgct ccaccctcct caagtagctt gctcgaagag 720

gatagtagct gcatcacatg gcttgataag caatctccta agtcagtcat ctatgttagc 780

tgggggagct tggcatgcat ggatgccaag gacctagctg aggttgcttg gggcctggcc 840

aacagcaacc agcctttctt gtgggtggtt cgacccggct cggtccgcgg ctcacagtgg 900

atagagcagc tgccggagac cttcatggac acagttggag aaagatgcca cattgtgaaa 960

tgggcacccc agaaggaagt gttggggcac cgagcagttg gagggttttg gagccattgt 1020

ggctggaact caacactgga aagcatctcc gaaggggttc caatgatctg caggccttat 1080

tcaggggacc aaagggtgaa cacaaggtat ataagccatg tttggaaggt gggattggag 1140

ttggagagtg atgagttaga gagggtggag atagaaagag ctgtgagaag gctgatggta 1200

gatggagaag gggaggagat gaggcagagg gcaatggaac tgaaggagaa ggttgatatt 1260

tgtacaagtg aaggtggttc ttccaacaga gccttgaagg agctggtgga gtatatctct 1320

tcattctag 1329

13